(19) Europäisches Patentamt
European Patent Office
Office européen des brevets

(11) EP 4 353 154 A1

(12) # EUROPEAN PATENT APPLICATION
published in accordance with Art. 153(4) EPC

(43) Date of publication:
**17.04.2024 Bulletin 2024/16**

(21) Application number: **22820013.5**

(22) Date of filing: **18.05.2022**

(51) International Patent Classification (IPC):
***A61B 5/256*** *(2021.01)*

(52) Cooperative Patent Classification (CPC):
**A61B 5/256**

(86) International application number:
**PCT/JP2022/020716**

(87) International publication number:
**WO 2022/259831 (15.12.2022 Gazette 2022/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.06.2021 JP 2021094959**

(71) Applicant: **Sumitomo Bakelite Co.Ltd.**
**Shinagawa-ku**
**Tokyo 140-0002 (JP)**

(72) Inventor: **ONO, Yoshiatsu**
**Tokyo 140-0002 (JP)**

(74) Representative: **Ter Meer Steinmeister & Partner**
**Patentanwälte mbB**
**Nymphenburger Straße 4**
**80335 München (DE)**

(54) **BRAIN WAVE MEASURING DEVICE AND BRAIN WAVE MEASURING METHOD**

(57) A brain wave measuring device (10) includes a band member (11) that is worn on a human head (99) by conforming to the shape of the human head, a plurality of electrode portions (13) that is provided on one surface of the band member (11), and conformance assisting portions (30) that assist the band member (11) in conforming to the shape of the head (99), in which each of the conformance assisting portions (30) has an ear attachment portion (40) that is attached to a human ear (80), an attachment portion (50) that is attached to the band member (11), and a connection member (60) that spans between the ear attachment portion (40) and the attachment portion (50).

FIG. 1

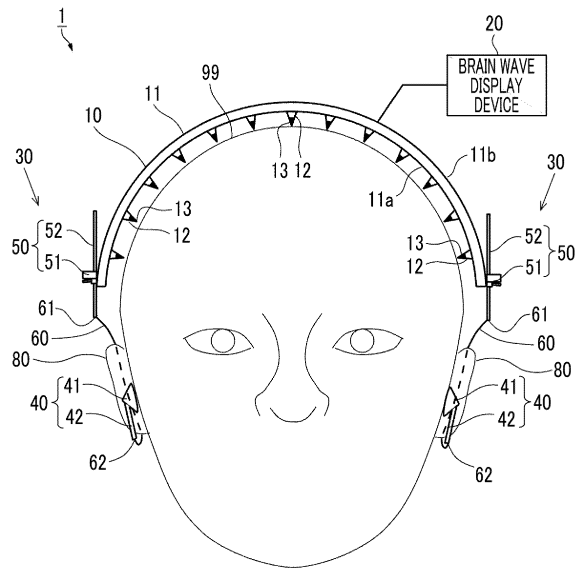


EP 4 353 154 A1

## Description

TECHNICAL FIELD

The present invention relates to a brain wave measuring device and a brain wave measuring method.

BACKGROUND ART

Various developments have been made in brain wave measuring devices. As this sort of technology, for example, a technology described in Patent Document 1 or 2 is known.
In the technique disclosed in Patent Document 1, a brain wave measuring device has a comb tooth array that is composed of a plurality of conductive comb teeth and enters between the hairs of a subject to reach the scalp.
In the technique disclosed in Patent Document 2, a brain wave measuring device is configured with a band to be worn on the head of a person, an adjustable first strip bonded to the band, electrodes of a first set bonded to the first strip to collect signals of the first set from the head, and a magnetic fastener for bonding the first strip to a band.

RELATED DOCUMENT

PATENT DOCUMENT

[Patent Document 1] Japanese Unexamined Patent Publication No. 2018-175287
[Patent Document 2] Japanese Unexamined Patent Publication No. 2018-94434

SUMMARY OF THE INVENTION

TECHNICAL PROBLEM

However, unfortunately, depending on the shape of head, brain wave detection electrodes cannot be worn on the head. In addition, even though the electrodes can be worn, sometimes pressure is brought to bear on the electrode portions, which may cause discomfort for some people. Particularly, excessive pressure is brought to bear on the electrode portions depending on the shape of head, which may cause extreme discomfort for some people. On the other hand, in a case where emphasis is placed on the ease of discomfort, the contact between the electrode portions and the head becomes insufficient, which unfortunately makes it difficult to appropriately measure brain waves. Because the techniques disclosed in Patent Documents 1 and 2 do not suggest solutions to the above problems, there have been demands for new techniques.
The present invention has been made in view of such a situation, and an object of the present invention is to provide a brain wave measuring device that is wearable regardless of the shape of head of a wearer and a brain wave measuring method.

SOLUTION TO PROBLEM

According to the present invention, the following techniques are provided.

[1] A brain wave measuring device having a band member that is worn on a human head by conforming to a shape of the human head,

a plurality of electrode portions that is provided on one surface of the band member, and
conformance assisting portions that assist the band member in conforming to the shape of the head,
in which each of the conformance assisting portions has
an ear attachment portion that is attached to a human ear,
an attachment portion that is attached to the band member, and
a connection member that spans between the ear attachment portion and the attachment portion.

[2] The brain wave measuring device described in [1], in which each of the conformance assisting portions has an adjustment portion that adjusts the way the band member conforms to the shape of the head.
[3] The brain wave measuring device described in [2], in which the adjustment portion has a mechanism that adjusts

a distance between the ear attachment portion and the attachment portion.

[4] The brain wave measuring device described in [3], in which the adjustment portion has a plate-shaped member and a lock portion that locks the plate-shaped member at a predetermined position.

[5] The brain wave measuring device described in any one of [1] to [4], in which the connection member has an elastic member.

[6] The brain wave measuring device described in any one of [1] to [5], in which the ear attachment portion is attached to concha by being fitted to the concha.

[7] The brain wave measuring device described in [6], in which in a state of being attached to the concha, the ear attachment portion has through-holes that connect inside and outside of the concha.

[8] The brain wave measuring device described in any one of [1] to [5], in which the ear attachment portion is attached by sandwiching an external ear portion.

[9] The brain wave measuring device described in any one of [1] to [5], in which the ear attachment portion is attached by being hooked around an external ear portion.

[10] The brain wave measuring device described in any one of [1] to [9], in which the band member is configured with a rubber-like elastic material.

[11] The brain wave measuring device described in any one of [1] to [10], further having a plurality of elastic protrusion portions integrated with the band member, in which the electrode portions are provided on the protrusion portions.

[12] The brain wave measuring device described in [11], in which each of the electrode portions has a conductive member provided on at least a tip portion of each of the protrusion portions.

[13] A brain wave measuring method for measuring brain waves by putting the brain wave measuring device described in any one of [1] to [12] on a subject's head.

ADVANTAGEOUS EFFECTS OF INVENTION

According to the present invention, it is possible to provide a brain wave measuring device that is wearable regardless of the wearer's head and a brain wave measuring method.

BRIEF DESCRIPTION OF THE DRAWINGS

Fig. 1 is a schematic view showing a brain wave detection system in a state where a brain wave measuring device is worn on a portion according to an embodiment.
Fig. 2 is a schematic view showing a brain wave detection system in a state where a brain wave measuring device is worn on a head according to an embodiment.
Fig. 3 is a front view of a brain wave detection electrodes according to an embodiment.
Fig. 4 is a plan view of brain wave detection electrodes according to an embodiment.
Fig. 5 is an enlarged view of a partial region X1 of Fig. 3 according to the embodiment.
Fig. 6 is a cross-sectional view taken along a line X2-X2 of Fig. 4 according to an embodiment.
Fig. 7 is a view showing another example of the cross-sectional view taken along the line X2-X2 of Fig. 4 according to an embodiment.
Fig. 8 is a plan view of another example (form 2) of brain wave detection electrodes according to an embodiment.
Fig. 9 is a plan view of another example (form 3) of brain wave detection electrodes according to an embodiment.
Fig. 10 is a plan view of another example (form 4) of brain wave detection electrodes according to an embodiment.
Fig. 11 is a plan view of another example (form 5) of brain wave detection electrodes according to an embodiment.
Fig. 12 is a plan view of another example (form 6) of brain wave detection electrodes according to an embodiment.
Fig. 13 is a plan view of another example (form 7) of brain wave detection electrodes according to an embodiment.
Fig. 14 is a plan view of another example (form 8) of brain wave detection electrodes according to an embodiment.
Fig. 15 is a plan view of another example (form 9) of brain wave detection electrodes according to an embodiment.
Fig. 16 is a plan view of another example (form 10) of brain wave detection electrodes according to an embodiment.
Figs. 17A and 17B are schematic views of an ear attachment portion according to an embodiment.
Fig. 18 is a view showing another example (form 2) of an ear attachment portion according to an embodiment.
Fig. 19 is a view showing another example (form 3) of an ear attachment portion according to an embodiment.
Fig. 20 is a view showing another example (form 4) of an ear attachment portion according to an embodiment.
Figs. 21A and 21B are views showing an attachment portion according to an embodiment.

DESCRIPTION OF EMBODIMENTS

Hereinafter, embodiments of the present invention will be described with reference to drawings.

The present embodiments will be described by defining front, rear, right, left, upper, and lower directions as shown in the drawings. However, the directions are defined for convenience of easy understanding of the relative relationship between constituents. Accordingly, this definition does not limit directions during the manufacturing or use of a product according to the present invention.

In all drawings, the same constituents will be marked with the same reference numerals, and the description thereof will not be repeated. In addition, the drawings are schematic views and do not consistent with the actual dimensional ratios.

Figs. 1 and 2 are schematic views showing a brain wave detection system 1 in a state where a brain wave measuring device 10 is worn on a head 99 of a person. Fig. 1 is a view of a wearer seen from the front. Fig. 2 is a view of the wearer seen from the side. The brain wave detection system 1 includes the brain wave measuring device 10 and a brain wave display device 20.

The brain wave measuring device 10 is worn on the head 99 of a person, detects brain waves as potential variation from a living body, and outputs the detected brain waves to the brain wave display device 20. The brain wave display device 20 obtains the brain waves detected by the brain wave measuring device 10 and displays the brain waves on a monitor, stores data, or performs a known brain wave analysis process (measurement process).

<Schematic structure of brain wave measuring device 10>

As shown in Fig. 1 or 2, the brain wave measuring device 10 includes a band member 11 as a rubber-like elastic material that is worn conforming to the shape of the head 99 of a human being, and conformance assisting portions 30 that assist the band member 11 in fitting (conforming) to the head 99 appropriately.

One surface of the band member 11 (in the present embodiment, a band inner surface 11a on the head 99 side) is provided with a plurality of elastic protrusion portions 12 integrated with the band member 11. At least the tip portion of each of the protrusion portions 12 configures each of the electrode portions 13 consisting of a conductive member.

The conformance assisting portions 30 are provided at both the longitudinal end portions of the band member 11, and each include an ear attachment portion 40 attached to an ear 80, an attachment portion 50 attached to the band member 11, and a connection portion 60 (connection member) that spans between the ear attachment portion 40 and the attachment portion 50.

The above configuration can prevent an incident where brain waves cannot be appropriately measured because both end portions of the band member 11 float above the head 99.

<Schematic configuration of system of brain wave display device 20>

The brain wave measuring device 10 includes a connector, an electronic component, and the like, and is connected to the brain wave display device 20. The brain wave measuring device 10 and the brain wave display device 20 may be integrated. In addition, the brain wave display device 20 may be configured with a smart device (a smartphone or a tablet device) and a predetermined application functioning on the smart device. In this case, the brain wave measuring device 10 has a communication function of wirelessly transmitting detected brain waves.

The brain wave display device 20 has, for example, a control portion, a storage portion, a user IF, an output portion, and a brain wave processing data-processing portion. These include an arithmetic unit such as CPU, a memory such as ROM or RAM, a storage device such as HDD or SSD, a monitor, a communication IF, and the like, converts the brain waves obtained from the brain wave measuring device 10 into a usable data format by a predetermined program, and performs a known brain wave analysis function.

<Specific configuration of brain wave measuring device 10>

Regarding the specific structure of the brain wave measuring device 10, first, the configurations of the band member 11 and the electrode portions 13 will be described, and then the configuration of the conformance assisting portion 30 will be described.

<Band member 11>

Fig. 3 is a front view of the band member 11. Fig. 4 is a plan view of the band member 11. In Figs. 3 and 4, the band member 11 curved in Fig. 1 is shown in a flat state. In the description of each drawing, for the sake of convenience,

the thickness direction of the band member 11 will be described as a Z direction (upward direction is +Z), the longitudinal direction of the rectangular shape will be described as an X direction (right direction is +X), and the short direction will be described as a Y direction (depth direction is +Y). In addition, the inner side (+Y side) will be described as front, and the front side (-Y side) will be described as rear. The variation of the band member 11 will be described in Figs. 8 to 16. For the sake of convenience, the band member 11 described in Figs. 1 to 7 will be regarded as form 1, and the band member 11 described in Figs. 8 to 16 will be regarded as form 2 to form 10.

<Shape of band member 11>

The band member 11 is a plate-like substance having a predetermined thickness t. Specifically, the band member 11 looks like a belt-shaped rectangle when seen in a top view (plan view).

The thickness t of the band member 11 is, for example, 0.1 mm to 30 mm.

A length L1 of the rectangle in the longitudinal direction is, for example, 20 cm to 65 cm.

A length L2 of the rectangle in the short direction is, for example, 0.5 cm to 5 cm.

The shape of the band member 11 is not limited to the belt-shaped rectangle. For example, the band member 11 may have the shape of a long and slender ellipse instead of the rectangular shape. In addition, the thickness t of the band member 11 is not limited to a constant thickness, and some portions of the band member 11 may be thinner or thicker. In any case, the band member 11 conforms to the shape of the head 99 in a case where the brain wave measuring device 10 is worn on the head 99.

<Arrangement of protrusion portions 12>

One surface (the band inner surface 11a on the head 99 side) of the band member 11 is provided with a plurality of protrusion portions 12 integrated with the band member 11. For example, as shown in the plan view of Fig. 4, the plurality of the protrusion portions 12 is arranged in a row at a predetermined pitch P when seen in a top view. The pitch P of the protrusion portions 12 (that is, the electrode portions 13) is, for example, 1 mm to 20 mm. The pitch P is determined from the viewpoint of the number of electrode portions 13 required for brain wave detection and the conformity of the band member 11 to the head 99.

<Shape of protrusion portions 12>

Figs. 5 and 6 are views showing the protrusion portions 12 seen from the front side (rear side). Fig. 5 is a view where the region X1 in the front view of Fig. 3 is enlarged to show one protrusion portion 12. Fig. 6 is a cross-sectional view taken along a line X2-X2 in Fig. 4, and is also a cross-sectional view of the protrusion portion 12 in Fig. 5.

The protrusion portions 12 are integrated with the band member 11 so as to protrude from one surface (herein, the band inner surface 11a) of the band member 11. The attachment portions 50 are attached to a surface (herein, a band outer surface 11b), the surface not being provided with the protrusion portions 12.

A height h1 of each protrusion portion 12 in the form of a trigonal pyramid is, for example, 0.5 mm to 20 mm, preferably 3 mm to 15 mm, and more preferably 4 mm to 10 mm.

Specifically, the trigonal pyramid as the shape of each protrusion portion 12 has, for example, an isosceles triangle as a base (that is, the boundary portion with the band inner surface 11a) having acute angles on vertices as shown in Fig. 4, and aligned in the same direction. Herein, the apex of the isosceles triangle faces one side (front side (+Y side) in the drawing) of the rectangular shape in the short direction, and the bottom side thereof faces the other side (rear side (-Y side) in the drawing). In addition, in the example shown in the drawing, the apex of the trigonal pyramid (that is, the tip of the protrusion portion 12) is located at the centroid of the isosceles triangle when seen in a top view. In other words, in the drawing, the protrusion portion 12 is in a direction such that it forms a gentle slope on the front side (+Y side) and has a steep surface on the rear side (-Y side). In the present embodiment, "the direction of the protrusion portion 12" means "direction that the apex of the isosceles triangle faces" described above.

When the brain wave measuring device 10 is worn on the head 99, by applying the protrusion portions 12 to the head 99 from the gentle side (+Y side), it is possible to smoothly put on the brain wave measuring device 10 without making the subject feel discomfort (such as pain) or the like and encountering less resistance from hair.

<Shape and material of electrode portions 13>

The electrode portions 13 consisting of a conductive member are provided in at least the tip portions of the protrusion portions 12 to cover the surface of the protrusion portions 12. Herein, each of the electrode portions 13 is provided on a surface ranging from the apex of the trigonal pyramid as the protrusion portion 12 to a predetermined height h2.

The predetermined height h2 of the electrode portion 13 to be formed depends on the height h1 of the protrusion portion 12, but is, for example, 1 mm to 10 mm.

The conductive member of the electrode portions 13 is, for example, a paste containing a good conductive metal. The good conductive metal includes one or more metals selected from the group consisting of copper, silver, gold, nickel, tin, lead, zinc, bismuth, antimony, and alloys of these. Particularly, from the viewpoint of availability and conductivity, silver, silver chloride, and copper are suitable.

To form the electrode portions 13 by using a paste containing a good conductive metal, the apex of the protrusion portions 12 formed of a rubber-like elastic material is dipped into a conductive solution in the form of a paste containing a good conductive metal (dip coating). In this way, the electrode portions 13 are formed on the surface of the tip portion of the protrusion portions 12.

The tip portion of the protrusion portions 12 may be coated with the conductive solution containing a conductive filler and a solvent, such that the electrode portions 13 as a conductive resin layer are formed. At this time, in a case where the same type of material (silicone rubber) as the protrusion portions 12 is used as the solvent, the adhesion of the electrode portions 13 (conductive resin layer) is improved.

A conductive signal line 14 connected to each electrode portion 13 is provided on the inside of each protrusion portion 12. The material, the thickness, and the arrangement position of the signal line 14 are not particularly limited as long as the brain waves can be appropriately measured with the connected brain wave display device 20 or the like. In a case where the electrode portion 13 is provided on the surface of the tip portion of the protrusion portion 12, for example, as shown in Fig. 6, the signal line 14 is connected to the inner surface of the electrode portion 13 on the apex portion of the protrusion portion 12 (that is, surface on the side connected to the protrusion portion 12).

A specific aspect of the signal line 14 suitable for the brain wave measuring device 10 of the present embodiment will be described below.

The signal line 14 is electrically connected to the electrode portion 13 covering the tip of the protrusion portion 12, and is disposed on the inside of the protrusion portion 12 toward the band member 11 from the tip.

As the signal line 14, known materials can be used. For example, the signal line 14 can be configured with conductive fiber.

As the conductive fiber, it is possible to use one or more fibers selected from the group consisting of metal fiber, metal-coated fiber, carbon fiber, conductive polymer fiber, conductive polymer-coated fiber, and conductive paste-coated fiber. Each of these fibers may be used alone, or two or more fibers described above may be used in combination.

The metal material of the metal fiber and the metal-coated fiber is not particularly limited as long as it has conductivity. Examples of the metal material include copper, silver, gold, nickel, tin, lead, zinc, bismuth, antimony, stainless steel, aluminum, silver/silver chloride, and alloys of these. Each of these materials may be used alone, or two or more materials described above may be used in combination. Among these, from the viewpoint of conductivity, silver can be used. In addition, it is preferable that the metal material do not include a metal such as chromium that imposes burden on the environment.

The fiber material of the metal-coated fiber, the conductive polymer-coated fiber, and the conductive paste-coated fiber is not particularly limited, and may be any of synthetic fiber, semisynthetic fiber, and natural fiber. Among these, polyester, nylon, polyurethane, silk, cotton, and the like are preferably used. Each of these materials may be used alone, or two or more materials described above may be used in combination.

Examples of the carbon fiber include a PAN-based carbon fiber and a pitch-based carbon fiber.

As the conductive polymer material of the conductive polymer fiber and the conductive polymer-coated fiber, for example, polythiophene, polypyrrole, polyaniline, polyacetylene, polyphenylene vinylene, polynaphthalene, a mixture of conductive polymers of derivatives of these and a binder resin, or an aqueous solution of a conductive polymer such as PEDOT-PSS ((3,4-ethylenedioxythiophene)-poly(styrene sulfonate)) is used.

The resin material contained in the conductive paste of the conductive paste-coated fiber is not particularly limited, and preferably has elasticity. For example, the resin material includes one or more materials selected from the group consisting of silicone rubber, urethane rubber, fluorine rubber, nitrile rubber, acrylic rubber, styrene rubber, chloroprene rubber, and ethylene propylene rubber. Each of these materials may be used alone, or two or more materials described above may be used in combination.

The conductive filler contained in the conductive paste of the conductive paste-coated fiber is not particularly limited, and a known conductive material may be used. For example, the conductive filler can include one or more materials selected from the group consisting of metal particles, metal fiber, metal-coated fiber, carbon black, acetylene black, graphite, carbon fiber, carbon nanotubes, a conductive polymer, conductive polymer-coated fiber, and metal nanowires.

The metal configuring the conductive filler is not particularly limited. For example, the metal may include at least one metal or two or more metals among copper, silver, gold, nickel, tin, lead, zinc, bismuth, antimony, silver/silver chloride, and alloys of these. Among these, in view of high conductivity and high availability, silver or copper is preferable.

The signal line 14 may be configured with spun yarn obtained by twisting a plurality of linear conductive fibers.

Using such spun yarn makes it possible to suppress disconnection of the signal line 14 during deformation.

In the present specification, coating of conductive fiber means not only covering of the outer surface of the fiber material. In the case of spun yarn obtained by twisting single fibers, the coating of conductive fiber also means that the voids of the fiber in the spun yarn are impregnated with a metal, a conductive polymer, or a conductive paste such that each of the single fibers configuring the spun yarn is coated.

The tensile elongation at break of the signal line 14 is, for example, 1% or more and 50% or less, and preferably 1.5% or more and 45% or less. In a case where the tensile elongation at break is in such a numerical range, it is possible to inhibit the protrusion portions 12 from being excessively deformed while suppressing break during deformation.

The signal line 14 can adopt various arrangement structures as long as it conducts electricity on the inside of the protrusion portions 12.

For example, the tip of the signal line 14 may have any of a structure protruding to the tip of the protrusion portion 12 or to the slope of the tip portion, a structure that is substantially in the same plane with the aforementioned tip or slope, and a structure buried in the aforementioned tip or slope. From the viewpoint of connection stability with the electrode portions 13, a protruding structure may be used. The protruding portion of the tip of the signal line 14 is partially or fully covered with the electrode portion 13.

For the protruding structure of the tip of the signal line 14, it is possible to adopt a structure with or without folds or a structure wrapped around the surface of the tip portion of the protrusion portion 12. In addition, the signal line 14 may incline to a vertical line extending from the tip (apex) of the protrusion portion 12 without coinciding with the vertical line.

As shown in the cross-sectional view of Fig. 7, the signal line 14 may be in the form of a line that is connected to an end portion on the lower side (on the band member 11 side) of the electrode portion 13, extends along the slope (surface) of the protrusion portion 12, and is drawn into the protrusion portion 12 from a predetermined position.

In the signal lines 14, each of the end portions opposite to the end portion connected to the electrode portion 13 may be pulled out to the outside of the band member 11. Furthermore, a plurality of signal lines 14 may be connected to a connector or the like provided on the band outer surface 11b of the band member 11 from the inside of the band member 11 and grouped.

Next, other forms (Forms 2 to 10) of the band member 11 will be described with reference to Figs. 8 to 16.

<Form 2 of band member 11>

Fig. 8 is a plan view of a band member 11A of form 2. The main difference between Form 2 and Form 1 is the arrangement of the protrusion portions 12. That is, in Form 1, the plurality of protrusion portions 12 is arranged in a row in the same direction.

On the other hand, in Form 2, the plurality of protrusion portions 12 is arranged in two rows. That is, it can be said that Form 2 is configured with one more row which is the array of the protrusion portions 12 of Form 1. In other words, the plurality of protrusion portions 12 is arranged in a grid shape (regular lattice shape).

In the band member 11A of Form 2, the plurality of protrusion portions 12 is arranged in a grid shape (regular lattice shape). Accordingly, the effect of Form 1 can be brought about, and the protrusion portions 12 (that is, the electrode portions 13) can be arranged in a way suitable for brain wave detection. That is, brain waves can be stably detected.

<Form 3 of band member 11>

Fig. 9 is a plan view of a band member 11B of Form 3. The main difference between Form 3 and Form 1 is the arrangement of the protrusion portions 12. That is, in Form 1, the plurality of protrusion portions 12 is arranged in a row in the same direction.

On the other hand, in Form 3, the plurality of protrusion portions 12 is arranged in two rows. Here, unlike in the band member 11A of Form 2, the protrusion portions 12 are in an alternate arrangement. In other words, the plurality of protrusion portions 12 is arranged in a herringbone lattice.

In the band member 11B of Form 3, the plurality of protrusion portions 12 is arranged in a herringbone lattice. Accordingly, the effect of Form 1 can be brought about, and the protrusion portions 12 (that is, the electrode portions 13) can be arranged in a way suitable for brain wave detection. That is, brain waves can be stably detected.

<Form 4 of band member 11>

Fig. 10 is a plan view of a band member 11C of Form 4. The present form is characterized in that the plurality of protrusion portions 12 is arranged in two rows, and that the number (density) of protrusion portions 12 in the row on the front side (+Y side row) has been reduced. Specifically, the protrusion portions 12 in the row on the rear side (-Y side row) in the drawing are arranged at a predetermined first pitch $P_{c1}$. In addition, the protrusion portions 12 in the row on the inner side (+Y side) are arranged at a predetermined second pitch $P_{c2}$ (for example, twice the first pitch $P_{c1}$). In

other words, it can also be said that the plurality of the protrusion portions 12 is arranged at different densities.

In the band member 11C of the present form, the plurality of protrusion portions 12 is in an alternate arrangement at different densities. Accordingly, the effect of Form 1 can be brought about, and the protrusion portions 12 (that is, the electrode portions 13) can be arranged in a way suitable for brain wave detection. That is, brain waves can be stably detected. Specifically, the shape of human head 99 is regular and common to people. In addition, there is a certain characteristic in the shape of the position of the head 99 on which the brain wave measuring device 10 (the band member 11C) is to be worn and the position of the head 99 that comes into contact with brain wave measuring device 10 when the device is worn. With the band member 11C, it is possible to excellently conform to such characteristics.

<Form 5 of band member 11>

Fig. 11 is a plan view of a band member 11D of Form 5. The band member 11D of the present form is the same as Form 1 in that the plurality of protrusion portions 12 is arranged in a row, but the direction of the protrusion portions 12 is different from that of Form 1. Therefore, the band member 11D appropriately conforms to the flow of hair.

Specifically, half the protrusion portions 12 on the left side in the drawing (8 protrusion portions 12 on the left side) face right (that is, face the longitudinal center of the band member 11), and half the protrusion portions 12 on the right side in the drawing (8 protrusion portions 12 on the right side) face left (that is, face the longitudinal center of the band member 11D). In other words, it can be said that each of the protrusion portions 12 face the apex of the head 99 when the brain wave measuring device 10 (band member 11D) is worn on the head 99.

In the band member 11D of the present form, each of the protrusion portions 12 faces the apex of the head 99 when the band member 11D is worn on the head 99 as described above. Therefore, the brain wave measuring device 10 (the band member 11D) can be worn appropriately conforming to the flow and amount of hair of the head 99. Particularly, in a case where the flat band member 11D is curved and worn as it is, the gentle side of the protrusion portions 12 comes into contact with the head 99, which makes it possible to avoid causing discomfort.

<Form 6 of band member 11>

Fig. 12 is a plan view of a band member 11E of Form 6. The band member 11E of the present form is the same as Forms 1 and 5 in that the plurality of protrusion portions 12 is arranged in a row, but the direction of the protrusion portions 12 is different from that of Forms 1 and 5. Therefore, the band member 11D appropriately conforms to the flow of hair.

Specifically, half the protrusion portions 12 on the left side in the drawing (8 protrusion portions 12 on the left side) face left (that is, face the left end portion of the band member 11 in the longitudinal direction), and half the protrusion portions 12 on the right side in the drawing (8 protrusion portions 12 on the right side) face right (that is, face the right end portion of the band member 11E in the longitudinal direction). In other words, it can be said that each of the protrusion portions 12 face the lower side (ear position side) from the apex of the head 99 when the brain wave measuring device 10 (band member 11E) is worn on the head 99.

In the band member 11E of the present form, each of the protrusion portions 12 faces lower side from the apex of the head 99 when the brain wave measuring device 10 (band member 11E) is worn on the head 99 as described above. Therefore, the brain wave measuring device 10 (the band member 11E) can be worn appropriately conforming to the flow and amount of hair of the head 99. Particularly, in a case where the band inner surface 11a of the band member 11E is curved to some extent and worn as it is, the gentle side of the protrusion portions 12 comes into contact with the head 99, which makes it possible to avoid causing discomfort.

<Form 7 of band member 11>

Fig. 13 is a plan view of a band member 11F of Form 7. In the band member 11F of the present form, in addition to the arrangement of the protrusion portions 12 in the band member 11D of Form 5, two protrusion portions 12 facing each other are further provided at the longitudinal center of the band member 11F.

Specifically, regarding the added two facing protrusion portions 12, the protrusion portion 12 on the front side (+Y side) in the drawing faces rear (-Y direction), and the protrusion portion 12 on the rear side (-Y side) in the drawing faces front (+ Y direction). That is, there are provided two facing protrusion portions 12 that face the center (center in each of the longitudinal and short directions) of the band member 11F (the band inner surface 11a) when seen in a top view.

The band member 11F of the present form brings about the same effect as the band member 11D of Form 5. Furthermore, the addition of two facing protrusion portions 12 described above enables the brain wave measuring device 10 (band member 11F) to be stably worn on the head 99.

<Form 8 of band member 11>

Fig. 14 is a plan view of a band member 11G of Form 8. In the band member 11G of the present form, in addition to the arrangement of the protrusion portions 12 in the band member 11E of Form 6, two protrusion portions 12 facing opposite sides are further provided at the longitudinal center of the band member 11G.

Specifically, regarding the added two protrusion portions 12 facing opposite sides, the protrusion portion 12 on the front side (+Y side) in the drawing faces front (+Y direction), and the protrusion portion 12 on the rear side (-Y side) in the drawing faces rear (-Y direction). That is, there are provided two facing protrusion portions 12 that face opposite sides at the center (center in each of the longitudinal and short directions) of the band member 11G (the band inner surface 11a) when seen in a top view.

The band member 11G of the present form brings about the same effect as the band member 11E of Form 6. Furthermore, the addition of two protrusion portions 12 facing opposite sides described above enables the brain wave measuring device 10 (band member 11G) to be more precisely worn on the head 99, particularly, can improve the function of making the protrusion portions 12 enter between hair and stabilized.

<Form 9 of band member 11>

In the band member 11H of Form 9, the row of the plurality of protrusion portions 12 in the band member 11D of Form 5 is changed to two rows, front and back rows. In other words, it can also be said that the characteristics of Form 2 and Form 5 are combined.

Specifically, in each of the front and back rows, half the protrusion portions 12 on the left side in the drawing (8 protrusion portions 12 on the left side) face right (that is, face the longitudinal center of the band member 11H), and half the protrusion portions 12 on the right side in the drawing (8 protrusion portions 12 on the right side) face left (that is, face the longitudinal center of the band member 11H)

The band member 11H of the present form brings about the same effect as the band member 11D of Form 5. Furthermore, arranging the plurality of protrusion portions 12 in two rows, front and back rows, can bring about the same effect as the band member 11B of Form 2, that is, enables the protrusion portions 12 (that is, the electrode portions 13) to be in an arrangement suitable for brain wave detection and makes it possible to stably detect brain waves.

<Form 10 of band member 11>

In the band member 11I of Form 10, the row of the plurality of protrusion portions 12 in the band member 11E of Form 6 is changed to two rows, front and back lines. In other words, it can also be said that the characteristics of Form 2 and Form 6 are combined.

Specifically, in each of the front and back rows, half the protrusion portions 12 on the left side in the drawing (8 protrusion portions 12 on the left side) face left (that is, face the left end portion of the band member 11E in the longitudinal direction), and half the protrusion portions 12 on the right side in the drawing (8 protrusion portions 12 on the right side) face right (that is, face the right end portion of the band member 11E in the longitudinal direction).

The band member 11I of the present form brings about the same effect as the band member 11E of Form 5. Furthermore, arranging the plurality of protrusion portions 12 in two rows, front and back rows, can bring about the same effect as the band member 11B of Form 2, that is, enables the protrusion portions 12 (that is, the electrode portions 13) to be in an arrangement suitable for brain wave detection and makes it possible to stably detect brain waves.

In Forms 1 to 10, the protrusion portions 12 have a trigonal pyramid shape for example. However, the protrusion portions 12 may be in the form of other pyramids such as conical and square pyramids, or in the form of a truncated pyramid formed by removing the apex of a pyramid.

<Materials of band member 11 and protrusion portions 12>

The band member 11 and the protrusion portions 12 are rubber-like elastic materials. More specifically, they are rubber or a thermoplastic elastomer (simply called "elastomer (TPE): as well). Examples of the rubber include silicone rubber. Examples of the thermoplastic elastomer include styrene-based TPE (TPS), olefin-based TPE (TPO), vinyl chloridebased TPE (TPVC), urethane-based TPE (TPU), ester-based TPE (TPEE), amide-based TPE (TPAE), and the like.

In a case where the band member 11 and the protrusion portions 12 of the brain wave measuring device 10 are silicone rubber, when the type A durometer hardness of the surface of the band member 11 (the band inner surface 11a or the band outer surface 11b) that is measured at 37°C based on JIS K 6253 (1997) is defined as a rubber hardness A, the rubber hardness A is, for example, 15 or more and 55 or less.

Next, the aforementioned silicone rubber-based curable composition will be described.

The silicone rubber can be configured with a cured substance of the silicone rubber-based curable composition. A step of curing the silicone rubber-based curable resin composition is performed, for example, by heating the composition at 100°C to 250°C for 1 to 30 minutes (primary curing) and post-baking the composition at 100°C to 200°C for 1 to 4 hours (secondary curing).

Insulating silicone rubber is silicone rubber not containing a conductive filler, and conductive silicone rubber is silicone rubber containing a conductive filler.

The silicone rubber-based curable composition according to the present embodiment may contain a vinyl group-containing organopolysiloxane (A). The vinyl group-containing organopolysiloxane (A) is a polymer containing the silicone rubber-based curable composition according to the present embodiment as a main element.

An insulating silicone rubber-based curable composition and a conductive silicone rubber-based curable composition may contain the same type of vinyl group-containing linear organopolysiloxanes. The same type of vinyl group-containing linear organopolysiloxanes may contain at least vinyl groups having the same functional groups and may be linear. The amount of vinyl groups in the molecule, the molecular weight distribution, or the addition amounts thereof may vary between the vinyl group-containing linear organopolysiloxanes.

The insulating silicone rubber-based curable composition and the conductive silicone rubber-based curable composition may further contain different types of vinyl group-containing organopolysiloxanes.

The vinyl group-containing organopolysiloxane (A) can contain a vinyl group-containing linear organopolysiloxane (A1) having a linear structure.

The vinyl group-containing linear organopolysiloxane (A1) has a linear structure and contains a vinyl group which functions as a crosslinking point during curing.

The content of the vinyl group in the vinyl group-containing linear organopolysiloxane (A1) is not particularly limited. For example, the vinyl group in the vinyl group-containing linear organopolysiloxane (A1) preferably has 2 or more vinyl groups in the molecule, and the content of the vinyl groups is preferably 15 mol% or less and more preferably 0.01 to 12 mol%. In a case where the content of the vinyl group is in the above range, the amount of the vinyl group in the vinyl group-containing linear organopolysiloxane (A1) can be optimized, and a network between the respective components, which will be described later, can be reliably formed. In the present embodiment, "to" means that the range includes numerical values as upper and lower limits.

In the present specification, the vinyl group content is mol% of a vinyl group-containing siloxane unit with respect to 100 mol% of all the units forming the vinyl group-containing linear organopolysiloxane (A1). Here, it is assumed that there may be one vinyl group for each vinyl group-containing siloxane unit.

The polymerization degree of the vinyl group-containing linear organopolysiloxane (A1) is not particularly limited and is, for example, preferably in a range of about 1,000 to 10,000 and more preferably in a range of about 2,000 to 5,000. The polymerization degree can be determined, for example, as a polystyrene-equivalent number-average polymerization degree (or number-average molecular weight) or the like gel permeation chromatography (GPC) using chloroform as an elution solvent.

The specific gravity of the vinyl group-containing linear organopolysiloxane (A1) is not particularly limited and is preferably in a range of about 0.9 to 1.1.

Using the vinyl group-containing linear organopolysiloxane (A1) having the polymerization degree and the specific gravity in the ranges described above makes it possible to obtain silicone rubber having higher heat resistance, higher flame retardancy, higher chemical stability, and the like.

It is preferable that the vinyl group-containing linear organopolysiloxane (A1) have a structure represented by the following Formula (1).

[Chem 1]

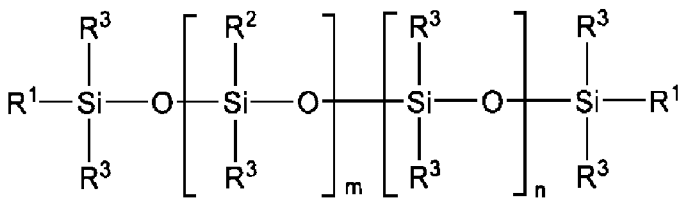

(1)

In Formula (1), $R^1$ represents a substituted or unsubstituted alkyl group, alkenyl group, or aryl group having 1 to 10 carbon atoms, or a hydrocarbon group as a combination of these. Examples of the alkyl group having 1 to 10 carbon atoms include a methyl group, an ethyl group, a propyl group, and the like. Among these, a methyl group is preferable. Examples of the alkenyl group having 1 to 10 carbon atoms include a vinyl group, an allyl group, a butenyl

group, and the like. Among these, a vinyl group is preferable. Examples of the aryl group having 1 to 10 carbon atoms include a phenyl group and the like.

$R^2$ represents a substituted or unsubstituted alkyl group, alkenyl group, or aryl group having 1 to 10 carbon atoms, or a hydrocarbon group as a combination of these. Examples of the alkyl group having 1 to 10 carbon atoms include a methyl group, an ethyl group, a propyl group, and the like. Among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

$R^3$ represents a substituted or unsubstituted alkyl group or aryl group having 1 to 8 carbon atoms, or a hydrocarbon group as a combination of these. Examples of the alkyl group having 1 to 8 carbon atoms include a methyl group, an ethyl group, a propyl group, and the like. Among these, a methyl group is preferable. Examples of the aryl group having 1 to 8 carbon atoms include a phenyl group.

Examples of substituents of $R^1$ and $R^2$ in Formula (1) include a methyl group, a vinyl group, and the like. Examples of substituents of $R^3$ include a methyl group and the like.

In Formula (1), a plurality of $R^1$'s is independent of each other, and may be the same as or different from each other. The same shall be applied to $R^2$ and $R^3$.

m and n each represent the number of repeating units configuring the vinyl group-containing linear organopolysiloxane (A1) represented by Formula (1). m represents an integer of 0 to 2,000, and n represents an integer of 1,000 to 10,000. m is preferably 0 to 1,000, and n is preferably 2,000 to 5,000.

Examples of specific structures of the vinyl group-containing linear organopolysiloxane (A1) represented by Formula (1) include a structure represented by the following Formula (1-1) .

[Chem 2]

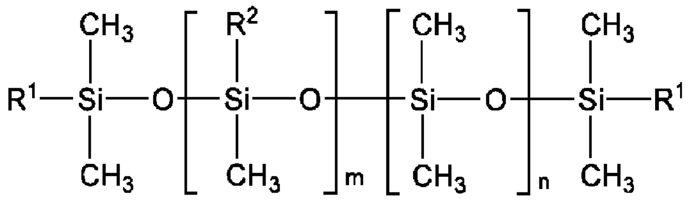

(1－1)

In Formula (1-1), $R^1$ and $R^2$ each independently represent a methyl group or a vinyl group, and at least one of $R^1$ or $R^2$ represents a vinyl group.

It is preferable that the vinyl group-containing linear organopolysiloxane (A1) contain a first vinyl group-containing linear organopolysiloxane (A1-1) that has two or more vinyl groups in the molecule and has a vinyl group content of 0.4 mol% or less and a second vinyl group-containing linear organopolysiloxane (A1-2) that has a vinyl group content of 0.5 to 15 mol%. Combining the first vinyl group-containing linear organopolysiloxane (A1-1) having the general vinyl group content and the second vinyl group-containing linear organopolysiloxane (A1-2) having a high vinyl group content as raw rubber that is a raw material of the silicone rubber enables vinyl groups to be dispersed, which makes it possible to more effectively create variations in the crosslinking density in the crosslinked network of the silicone rubber. As a result, the tear strength of the silicone rubber can be more effectively improved.

Specifically, as the vinyl group-containing linear organopolysiloxane (A1), for example, it is preferable to use the first vinyl group-containing linear organopolysiloxane (A1-1) which has two or more units represented by Formula (1-1) where either or both of $R^1$ and $R^2$ represent a vinyl group in the molecule and in which the content of such units is 0.4 mol% or less and the second vinyl group-containing linear organopolysiloxane (A1-2) in which the content of a unit represented by Formula (1-1) where either or both of $R^1$ and $R^2$ represent a vinyl group is 0.5 to 15 mol%.

The first vinyl group-containing linear organopolysiloxane (A1-1) preferably has a vinyl group content of 0.01 to 0.2 mol%. In addition, the second vinyl group-containing linear organopolysiloxane (A1-2) preferably has a vinyl group content of 0.8 to 12 mol%.

In a case where the first vinyl group-containing linear organopolysiloxane (A1-1) and the second vinyl group-containing linear organopolysiloxane (A1-2) are mixed together, the ratio between (A1-1) and (A1-2) is not particularly limited. For example, the weight ratio (A1-1):(A1-2) is preferably 50:50 to 95:5, and more preferably 80:20 to 90:10.

As each of the first and second vinyl group-containing linear organopolysiloxanes (A1-1) and (A1-2), only one compound may be used, or two or more compounds may be used in combination.

The vinyl group-containing organopolysiloxane (A) may contain a vinyl group-containing branched organopolysiloxane (A2) having a branched structure.

<<Organohydrogen Polysiloxane (B)>>

The silicone rubber-based curable composition of the present embodiment may contain a crosslinking agent. The crosslinking agent can contain an organohydrogen polysiloxane (B) .

The organohydrogen polysiloxane (B) is classified into a linear organohydrogen polysiloxane (B1) having a linear structure and a branched organohydrogen polysiloxane (B2) having a branched structure, and can include either or both of these.

The insulating silicone rubber-based curable composition and the conductive silicone rubber-based curable composition may contain the same type of crosslinking agents. The same type of crosslinking agents just need to have at least a common structure such as a linear structure or a branched structure. The crosslinking agents may have different molecular weight distributions in the molecule or may contain different functional groups, and the addition amounts thereof may be different.

The insulating silicone rubber-based curable composition and the conductive silicone rubber-based curable composition may further contain different types of crosslinking agents.

The linear organohydrogen polysiloxane (B1) is a polymer that has a linear structure and a structure (=Si-H) in which hydrogen is directly bonded to Si, and has a hydrosilylation reaction with vinyl groups contained in the components mixed with the silicone rubber-based curable composition in addition to the vinyl groups of the vinyl group-containing organopolysiloxane (A) to crosslink the components.

The molecular weight of the linear organohydrogen polysiloxane (B1) is not particularly limited. For example, the weight-average molecular weight thereof is preferably 20,000 or less, and more preferably 1,000 or more and 10,000 or less.

The weight-average molecular weight of the linear organohydrogen polysiloxane (B1) can be measured as a polystyrene-equivalent value by gel permeation chromatography (GPC) using chloroform as an elution solvent.

It is preferable that the linear organohydrogen polysiloxane (B1) do not have a vinyl group in general. In a case where the linear organohydrogen polysiloxane (B1) does not have a vinyl group, it is possible to reliably prevent the occurrence of a crosslinking reaction in the molecule of the linear organohydrogen polysiloxane (B1).

As the linear organohydrogen polysiloxane (B1), for example, a compound having a structure represented by the following Formula (2) is preferably used.

[Chem 3]

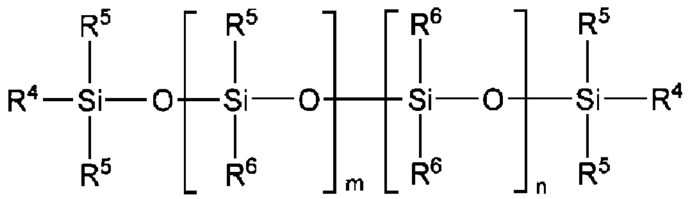

(2)

In Formula (2), $R^4$ represents a substituted or unsubstituted alkyl group, alkenyl group, or aryl group having 1 to 10 carbon atoms, a hydrocarbon group as a combination of these, or a hydride group. Examples of the alkyl group having 1 to 10 carbon atoms include a methyl group, an ethyl group, a propyl group, and the like. Among these, a methyl group is preferable. Examples of the alkenyl group having 1 to 10 carbon atoms include a vinyl group, an allyl group, a butenyl group, and the like. Examples of the aryl group having 1 to 10 carbon atoms include a phenyl group.

$R^5$ represents a substituted or unsubstituted alkyl group, alkenyl group, or aryl group having 1 to 10 carbon atoms, a hydrocarbon group as a combination of these, or a hydride group. Examples of the alkyl group having 1 to 10 carbon atoms include a methyl group, an ethyl group, and a propyl group. Among these, a methyl group is preferable. Examples of the alkenyl group having 1 to 10 carbon atoms include a vinyl group, an allyl group, a butenyl group, and the like. Examples of the aryl group having 1 to 10 carbon atoms include a phenyl group.

In Formula (2), a plurality of $R^4$'s is independent of each other, and may be the same as or different from each other. The same shall be applied to $R^5$. Here, at least two or more among the plurality of R4's and the plurality of $R^5$'s represent hydride groups.

R6 represents a substituted or unsubstituted alkyl group or aryl group having 1 to 8 carbon atoms, or a hydrocarbon group as a combination of these. Examples of the alkyl group having 1 to 8 carbon atoms include a methyl group, an ethyl group, a propyl group, and the like. Among these, a methyl group is preferable. Examples of the aryl group having 1 to 8 carbon atoms include a phenyl group. A plurality of $R^6$'s is independent of each other, and may be the same as or different from each other.

Examples of substituents of $R^4$, $R^5$, and $R^6$ in Formula (2) include a methyl group, a vinyl group, and the like. From the viewpoint of preventing an intramolecular crosslinking reaction, a methyl group is preferable.

m and n each independently represent the number of repeating units configuring the linear organohydrogen polysiloxane (B1) represented by Formula (2), m represents an integer of 2 to 150, and n represents an integer of 2 to 150. It is preferable that m represent an integer of 2 to 100 and n represent an integer of 2 to 100.

As the linear organohydrogen polysiloxane (B1), only one compound may be used alone, or two or more compounds may be used in combination.

Having a branched structure, the branched organohydrogen polysiloxane (B2) is a component that largely contributes to the formation of a density-varying crosslinking structure in the silicone rubber system by forming a region having a high crosslinking density. In addition, just as the linear organohydrogen polysiloxane (B1), the branched organohydrogen polysiloxane (B2) is a polymer that has a structure ( ≡Si-H) in which hydrogen is directly bonded to Si, and has a hydrosilylation reaction with vinyl groups of components mixed with the silicone rubber-based curable composition in addition to the vinyl groups of the vinyl group-containing organopolysiloxane (A) to crosslink these components.

The specific gravity of the branched organohydrogen polysiloxane (B2) is in a range of 0.9 to 0.95.

It is preferable that the branched organohydrogen polysiloxane (B2) do not have a vinyl group in general. In a case where the branched organohydrogen polysiloxane (B2) does not have a vinyl group, it is possible to reliably prevent the occurrence of a crosslinking reaction in the molecule of the linear organohydrogen polysiloxane (B2).

The branched organohydrogen polysiloxane (B2) is preferably represented by the following Average Compositional Formula (c).

Average Compositional Formula (c)

$$(H_a(R^7)_{3-a}SiO_{1/2})_m(SiO_{4/2})_n$$

(In Formula (c), $R^7$ represents a monovalent organic group, a represents an integer of 1 to 3, m represents the number of $H_a(R^7)_{3-a}SiO_{1/2}$ units, and n represents the number of $SiO_{4/2}$ units.)

In Formula (c), $R^7$ represents a monovalent organic group, and preferably represents a substituted or unsubstituted alkyl group or aryl group having 1 to 10 carbon atoms, or a hydrocarbon group as a combination of these. Examples of the alkyl group having 1 to 10 carbon atoms include a methyl group, an ethyl group, a propyl group, and the like. Among these, a methyl group is preferable. Examples of the aryl group having 1 to 10 carbon atoms include a phenyl group.

In Formula (c), a represents the number of hydride groups (hydrogen atoms directly bonded to Si) which is an integer of 1 to 3. a is preferably 1.

In addition, in Formula (c), m represents the number of $H_a(R^7)_{3-a}SiO_{1/2}$ units, and n represents the number of $SiO_{4/2}$ units.

The branched organohydrogen polysiloxane (B2) has a branched structure. The difference between the linear organohydrogen polysiloxane (B1) and the branched organohydrogen polysiloxane (B2) is whether the structure thereof is linear or branched. Assuming that the number of Si is 1, the number of alkyl groups R bonded to Si is in a range of 1.8 to 2.1 in the linear organohydrogen polysiloxane (B1) and is in a range of 0.8 to 1.7 in the branched organohydrogen polysiloxane (B2).

Having a branched structure, the branched organohydrogen polysiloxane (B2) leaves 5% or more residues in a case where the compound is heated, for example, to 1,000°C at a heating rate of 10 °C/min in a nitrogen atmosphere. On the other hand, having a linear structure, the linear organohydrogen polysiloxane (B1) substantially does not leave residues in a case where the compound is heated under the above conditions.

Specific examples of the branched organohydrogen polysiloxane (B2) include compounds having a structure represented by the following Formula (3).

[Chem 4]

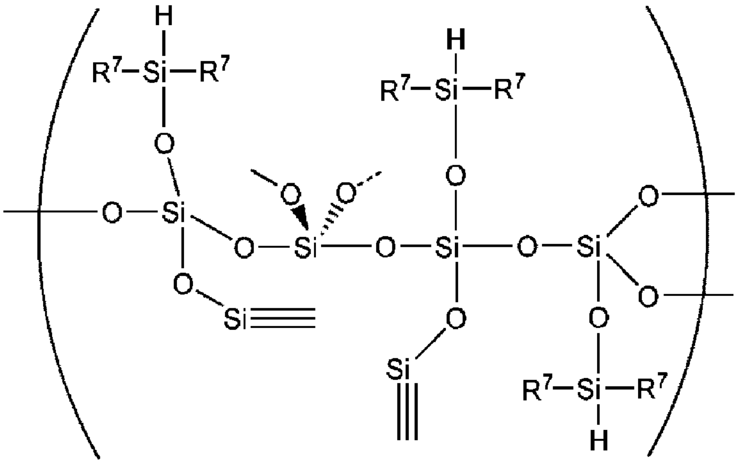

(3)

In Formula (3), $R^7$ represents a substituted or unsubstituted alkyl group or aryl group having 1 to 8 carbon atoms, a hydrocarbon group as a combination of these, or a hydrogen atom. Examples of the alkyl group having 1 to 8 carbon atoms include a methyl group, an ethyl group, a propyl group, and the like. Among these, a methyl group is preferable. Examples of the aryl group having 1 to 8 carbon atoms include a phenyl group. Examples of substituents of $R^7$ include a methyl group and the like.

In Formula (3), a plurality of $R^7$'s is independent of each other, and may be the same as or different from each other.

In Formula (3), "-O-Si≡" means that Si has a branched structure that expands three-dimensionally.

As the branched organohydrogen polysiloxane (B2), only one compound may be used alone, or two or more compounds may be used in combination.

In each of the linear organohydrogen polysiloxane (B1) and the branched organohydrogen polysiloxane (B2), the amount of hydrogen atoms (hydride groups) directly bonded to Si is not particularly limited. Here, in the silicone rubber-based curable composition, the total amount of hydride groups in the linear organohydrogen polysiloxane (B1) and the branched organohydrogen polysiloxane (B2) is preferably 0.5 to 5 mol and more preferably 1 to 3.5 mol with respect to 1 mol of vinyl groups in the vinyl group-containing linear organopolysiloxane (A1). In a case where the total amount of hydride groups is in the above range, a crosslinked network can be reliably formed among the linear organohydrogen polysiloxane (B1) and the branched organohydrogen polysiloxane (B2), and the vinyl group-containing linear organopolysiloxane (A1).

<<Silica particles (C)>>

The silicone rubber-based curable composition of the present embodiment contains a non-conductive filler. As necessary, the non-conductive filler may contain silica particles (C). In a case where the silicone rubber-based curable composition contains the silica particles (C), the hardness or mechanical strength of the elastomer can be improved.

The insulating silicone rubber-based curable composition and the conductive silicone rubber-based curable composition may contain the same type of non-conductive fillers. The same type of non-conductive fillers just need to have at least a common constituent material, and the particle size, specific surface area, surface treatment agent, or the addition amount thereof may vary between the fillers.

The insulating silicone rubber-based curable composition and the conductive silicone rubber-based curable composition may further contain different types of silane coupling agents.

The silica particles (C) are not particularly limited. For example, fumed silica, sintered silica, precipitated silica, or the like is used. Each of these materials may be used alone, or two or more materials described above may be used in combination.

The specific surface area of the silica particles (C) measured using, for example, a BET method is, for example, preferably 50 to 400 $m^2$/g, and more preferably 100 to 400 $m^2$¥g. The average primary particle size of the silica particles (C) is, for example, preferably 1 to 100 nm and more preferably about 5 to 20 nm.

Using the silica particles (C) having the specific surface area and the average particle size in the above ranges makes it possible to improve the hardness and the mechanical strength of the formed silicone rubber and, particularly, to improve the tensile strength of the formed silicone rubber.

<<Silane coupling agent (D)>

The silicone rubber-based curable composition according to the present embodiment may contain a silane coupling agent (D).

The silane coupling agent (D) may have a hydrolyzable group. The hydrolyzable group is hydrolyzed into a hydroxyl group by water. The hydroxyl group has a dehydration condensation reaction with a hydroxyl group on the surface of the silica particles (C), which can modify the surface of the silica particles (C).

The insulating silicone rubber-based curable composition and the conductive silicone rubber-based curable composition may contain the same type of silane coupling agents. The same type of silane coupling agents just need to have at least a common functional group and may have other functional groups in the molecule, and the addition amounts thereof may be different.

The insulating silicone rubber-based curable composition and the conductive silicone rubber-based curable composition may further contain different types of silane coupling agents.

The silane coupling agent (D) can include a silane coupling agent having a hydrophobic group. In this case, the hydrophobic group is added to the surfaces of the silica particles (C). Accordingly, in the silicone rubber-based curable composition and the silicone rubber, the cohesive force between the silica particles (C) decreases (cohesion through a hydrogen bond formed by a silanol group weakens). Presumably, as a result, the dispersibility of the silica particles (C) in the silicone rubber-based curable composition may be improved. Consequently, the interface between the silica particles (C) and a rubber matrix enlarges, and a reinforcing effect of the silica particles (C) increases. Furthermore, presumably, the silica particles (C) in the matrix may become more slippery during the matrix deformation of rubber. The improvement of dispersibility and slipperiness of the silica particles (C) leads to the improvement of the mechanical strength (for example, tensile strength, tear strength, or the like) of the silicone rubber by the silica particles (C).

The silane coupling agent (D) may include a silane coupling agent having a vinyl group. In a case where the silane coupling agent has a vinyl group, the vinyl group is introduced into the surface of the silica particles (C). Therefore, in a case where the silicone rubber-based curable composition is cured, that is, in a case where the vinyl groups of the vinyl group-containing organopolysiloxane (A) and the hydride groups in the organohydrogen polysiloxane (B) have a hydrosilylation reaction to form a network (crosslinked structure), the vinyl groups of the silica particles (C) are also involved in the hydrosilylation reaction with the hydride groups of the organohydrogen polysiloxane (B). As a result, the silica particles (C) are also incorporated into the network. Accordingly, it is possible to achieve the reduction in hardness and the increase in modulus of the formed silicone rubber.

As the silane coupling agent (D), a silane coupling agent having a hydrophobic group and a silane coupling agent having a vinyl group can be used in combination.

Examples of the silane coupling agent (D) include a compound represented by the following Formula (4).

$$Y_n\text{-}Si\text{-}(X)_{4-n} \quad (4)$$

In Formula (4), n represents an integer of 1 to 3. Y represents any functional group among a hydrophobic group, a hydrophilic group, and a vinyl group. When n represents 1, Y represents a hydrophobic group. When n represents 2 or 3, at least one of Y's represents a hydrophobic group. X represents a hydrolyzable group.

The hydrophobic group is an alkyl group or aryl group having 1 to 6 carbon atoms, or a hydrocarbon group as a combination of these. Examples of the hydrophobic group include a methyl group, an ethyl group, a propyl group, a phenyl group, and the like. Among these, a methyl group is particularly preferable.

Examples of the hydrophilic group include a hydroxyl group, a sulfonate group, a carboxyl group, a carbonyl group, and the like. Among these, a hydroxyl group is particularly preferable. The hydrophilic group may be incorporated into the silane coupling agent as a functional group. From the viewpoint of making the silane coupling agent (D) hydrophobic, it is preferable that the silane coupling agent (D) do not contain a hydrophilic group.

Examples of the hydrolyzable group include an alkoxy group such as a methoxy group or an ethoxy group, a chloro group, a silazane group, and the like. Among these, in view of high reactivity with the silica particles (C), a silazane group is preferable. Owing to its structural characteristics, the silane coupling agent having a silazane group as a hydrolyzable group has two structures represented by ($Y_n$-Si-) in Formula (4).

Specific examples of the silane coupling agent (D) represented by Formula (4) are as follows.

Examples of the silane coupling agent having a hydrophobic group as the functional group include an alkoxysilane such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, or decyltrimethoxysilane; a chlorosilane such as methyltrichlorosilane, dimethyldichlorosilane, trimethylchlorosilane, or phenyltrichlorosilane; and hexamethyldisilazane. Among these, a silane coupling agent having a trimethylsilyl group that includes one or more compounds selected from the group consisting of hexamethyldisilazane,

trimethylchlorosilane, trimethylmethoxysilane, and trimethylethoxysilane is preferable.

Examples of the silane coupling agent having a vinyl group as the functional group include an alkoxysilane such as methacryloxypropyltriethoxysilane, methacryloxypropyltrimethoxysilane, methacryloxypropylmethyldiethoxysilane, methacryloxypropylmethyldimethoxysilane, vinyltriethoxysilane, vinyltrimethoxysilane, or vinylmethyldimethoxysilane; a chlorosilane such as vinyltrichlorosilane or vinylmethyldichlorosilane; and divinyltetramethyldisilazane. Among these, a silane coupling agent having a vinyl group-containing organosilyl group that includes one or more compounds selected from the group consisting of methacryloxypropyltriethoxysilane, methacryloxypropyltrimethoxysilane, methacryloxypropylmethyldiethoxysilane, methacryloxypropylmethyldimethoxysilane, divinyltetramethyldisilazane, vinyltriethoxysilane, vinyltrimethoxysilane, and vinylmethyldimethoxysilane is preferable.

In a case where the silane coupling agent (D) includes two silane coupling agents, a silane coupling agent having a trimethylsilyl group and a silane coupling agent having a vinyl group-containing organosilyl group, it is preferable that the silane coupling agent (D) include hexamethyldisilazane as a silane coupling agent having a hydrophobic group and divinyltetramethyldisilazane as a silane coupling agent having a vinyl group.

In a case where a silane coupling agent (D1) having a trimethylsilyl group and a silane coupling agent (D2) having a vinyl group-containing organosilyl group are used in combination, a ratio between (D1) and (D2) is not particularly limited. For example, a weight ratio (D1):(D2) is 1:0.001 to 1:0.35, preferably 1:0.01 to 1:0.20, and more preferably 1:0.03 to 1:0.15. Setting the ratio to the numerical range described above makes it possible to obtain desired physical properties of silicone rubber. Specifically, the dispersibility of silica in the rubber and the crosslinking properties of the rubber can be balanced.

In the present embodiment, the lower limit of the content of the silane coupling agent (D) with respect to the total amount of 100 portions by weight of the vinyl group-containing organopolysiloxane (A) is preferably 1% by mass or more, more preferably 3% by mass or more, and even more preferably 5% by mass or more. Furthermore, the upper limit of the content of the silane coupling agent (D) with respect to the total amount of 100 portions by weight of the vinyl group-containing organopolysiloxane (A) is preferably 100% by mass or less, more preferably 80% by mass or less, and even more preferably 40% by mass or less.

Setting the content of the silane coupling agent (D) to be equal to or more than the aforementioned lower limit makes it possible to improve the adhesion between a columnar portion containing an elastomer and the conductive resin layer. In addition, the mechanical strength of the silicone rubber can be improved. Furthermore, setting the content of the silane coupling agent (D) to be equal to or less than the aforementioned upper limit makes it possible to obtain silicone rubber having appropriate mechanical characteristics.

<<Platinum or platinum compound (E)>>

The silicone rubber-based curable composition according to the present embodiment may contain a catalyst. The catalyst can include platinum or platinum compound (E). The platinum or platinum compound (E) is a catalyst component that functions as a catalyst during curing. The addition amount of the platinum or platinum compound (E) is the amount of the catalyst.

The insulating silicone rubber-based curable composition and the conductive silicone rubber-based curable composition may contain the same type of catalysts. The same type of catalysts just need to have at least a common constituent material. Different compositions may be contained in the catalysts, or the addition amounts thereof may vary between the catalysts.

The insulating silicone rubber-based curable composition and the conductive silicone rubber-based curable composition may further contain different catalysts.

As the platinum or platinum compound (E), known platinum or platinum compounds can be used. Examples thereof include platinum black, silica or carbon black on which platinum is supported, platinic chloride or an alcohol solution of platinic chloride, a complex salt of platinic chloride and olefin, a complex salt of platinic chloride and vinylsilxoane, and the like.

As the platinum or platinum compound (E), only one platinum element or one platinum compound may be used alone, or two or more platinum elements or two platinum compounds may be used in combination.

In the present embodiment, the content of the platinum or platinum compound (E) in the silicone rubber-based curable composition refers to the amount of the catalyst and can be appropriately set. Specifically, the content of platinum group metals by weight with respect to the total amount of 100 portions by weight of the vinyl group-containing organopolysiloxane (A), the silica particles (C), and the silane coupling agent (D) is 0.01 to 1000 ppm and preferably 0.1 to 500 ppm.

Setting the content of the platinum or platinum compound (E) to be equal to or more than the aforementioned lower limit makes it possible to cure the silicone rubber-based curable composition at an appropriate rate. In addition, setting the content of the platinum or platinum compound (E) to be equal to or less than the aforementioned upper limit makes it possible to reduce manufacturing costs.

<<Water (F)>>

The silicone rubber-based curable composition according to the present embodiment may contain water (F) in addition to the components (A) to (E).

The water (F) is a component that functions as a dispersion medium for dispersing the respective components in the silicone rubber-based curable composition and contributes to the reaction between the silica particles (C) and the silane coupling agent (D). Therefore, the silica particles (C) and the silane coupling agent (D) can be more reliably linked to each other in the silicone rubber, and the silicone rubber can show uniform characteristics as a whole.

(Other Components)

Further, the silicone rubber-based curable composition according to the present embodiment may further contain other components in addition to the components (A) to (F). Examples of those other components include an inorganic filler other than the silica particles (C), such as diatomaceous earth, iron oxide, zinc oxide, titanium oxide, barium oxide, magnesium oxide, cerium oxide, calcium carbonate, magnesium carbonate, zinc carbonate, glass wool, or mica, and an additive such as a reaction inhibitor, a dispersant, a pigment, a dye, an antistatic agent, an antioxidant, a flame retardant, or a thermal conductivity enhancing agent.

The conductive solution (conductive silicone rubber composition) according to the present embodiment contains the aforementioned conductive filler and a solvent in addition to the silicone rubber-based curable composition that does not contain a conductive filler.

As the solvent, various known solvents can be used. For example, the solvent can include a high boiling point solvent. Each of these solvents may be used alone, or two or more solvents described above may be used in combination.

Examples of the solvent include aliphatic hydrocarbons such as pentane, hexane, cyclohexane, heptane, methylcyclohexane, ethylcyclohexane, octane, decane, dodecane, and tetradecane; aromatic hydrocarbons such as benzene, toluene, ethylbenzene, xylene, trifluoromethylbenzene, and benzotrifluoride; ethers such as diethyl ether, diisopropyl ether, dibutyl ether, cyclopentyl methyl ether, cyclopentyl ethyl ether, ethylene glycol dimethyl ether, ethylene glycol diethyl ether, diethylene glycol dimethyl ether, 1,4-dioxane, 1,3-dioxane, and tetrahydrofuran; haloalkanes such as dichloromethane, chloroform, 1,1-dichloroethane, 1,2-dichloroethane, 1,1,1-trichloroethane, and 1,1,2-trichloroethane; carboxylic acid amides such as N,N-dimethylformamide and N,N-dimethylacetamide; sulfoxides such as dimethyl sulfoxide and diethyl sulfoxide, and the like. Each of these compounds may be used alone, or two or more compounds described above may be used in combination.

Adjusting the solid content or the like of the conductive solution enables the solution to have viscosity appropriate for various coating methods such as spray coating and dip coating.

In a case where the conductive solution contains the conductive filler and the silica particles (C), the lower limit of the content of the silica particles (C) contained in the electrode portions 13 with respect to the total amount of 1000 by mass of the silica particles (C) and the conductive filler is, for example, 1% by mass or more, preferably 3% by mass or more, and more preferably 5% by mass or more. In a case where the lower limit is in the above range, the mechanical strength of the electrode portions 13 can be improved. On the other hand, the upper limit of the content of the silica particles (C) contained in the electrode portions 13 with respect to the total amount of 100% by mass of the silica particles (C) and the conductive filler is, for example, 20% by mass or less, preferably 15% by mass or less, and more preferably 10% by mass or less. In a case where the upper limit is in the above range, conductivity and mechanical strength or flexibility in the electrode portions 13 can be balanced.

By heating and drying the conductive solution as necessary, the conductive silicone rubber is obtained.

The conductive silicone rubber may be configured such that it does not contain silicone oil. In a case where the conductive silicone rubber is configured as above, it is possible to prevent a phenomenon where silicone oil bleeds out to the surface of the electrode portions 13 and reduces conductivity.

By the above configuration, excellent conformity to the head 99 and desired strength can be simultaneously achieved. In addition, it is possible to avoid discomfort that may be caused by the protrusion portions 12 when the brain wave measuring device 10 is worn. As a result, brain wave can be stably detected. Note that the rubber hardness A may be selected according to the characteristics of the subject to wear the brain wave measuring device 10, for example, whether the subject is an "adult" or a "kid", whether the subject has sensitive scalp, whether the subject’s hair is hard or soft, and the like.

In a case where the band member 11 is formed of silicone rubber, by molding a curable elastomer composition such as a silicone rubber-based curable composition, a molded product consisting of a plurality of protrusion portions 12 and the band member 11 that are seamlessly bonded to each other is obtained. As a result, it is possible to obtain the brain wave measuring device 10 that is excellent in flexibility (that is, flexibility that can conform to the head 99) and strength and excellently conform to the head 99. The rubber hardness A, that is, the flexibility can be controlled by appropriately selecting the type or mixing amount of each component contained in the silicone rubber-based curable

composition, a method of preparing the silicone rubber-based curable composition, and the like.

<Manufacturing method of band member 11>

An example of a manufacturing method of the band member 11 can include the following steps.

First, the silicone rubber-based curable composition is subjected to hot press molding using a mold to obtain a molded product consisting of the band member 11 and the protrusion portions 12. Subsequently, by using a sewing needle, the signal line 14 is inserted into each columnar portion of the obtained molded product. Then, the surface (predetermined height h2) of the tip portion of the protrusion portions 12 of the obtained molded product is dip-coated with a paste-like conductive solution, and heated to dry, followed by post-curing. In this way, the electrode portions 13 can be formed on the surface of the protrusion portions 12.

Through the above steps, the band member 11 can be manufactured.

Note that in the aforementioned molding step, insert molding may be used which is a process of introducing the silicone rubber-based curable composition into a molding space where the signal line 14 is placed, followed by hot press molding.

<Conformance assisting portion 30>

Each conformance assisting portion 30 has an ear attachment portion 40, an attachment portion 50, and a connection portion 60. The ear attachment portion 40 is attached to a human ear 80. The attachment portion 50 is attached to the band member 11. The connection portion 60 is a wire having one end portion 61 that is attached to the attachment portion 50 and the other end portion 62 that is attached to the ear attachment portion 40, and spans between the ear attachment portion 40 and the attachment portion 50.

<Ear attachment portion 40>

Figs. 17A and 17B shows the ear attachment portion 40. Fig. 17A is a plan view of the ear attachment portion 40, and Fig. 17B is a side view. The ear attachment portion 40 has the same shape, for example, as a so-called open-ear type earphone, and is attached to ear to fit into the tympanic cavity 81. The ear attachment portion 40 may be provided with a member that fits to the external ear canal from an attachment portion body 41 just as a canal-type earphone.

The ear attachment portion 40 has the attachment portion body 41 that is attached to the tympanic cavity 81, and an extending portion 42 that extends from the attachment portion body 41 and extends outside the ear 80 (external ear) through an intertragic notch 84.

The attachment portion body 41 and the extending portion 42 are formed, for example, of hard members such as polyamide resins. However, the attachment portion body 41 and the extending portion 42 are not limited to these materials, and just need to be formed of materials capable of being attached to the ear 80 and staying attached to the ear. In addition, the attachment portion body 41 or the extending portion 42 does not need to be made of the same material as a whole. For example, the portion of the attachment portion body 41 that comes into contact with the tympanic cavity 81 may be a rubber member.

The attachment portion body 41 has a substantially conical shape, and a portion corresponding to a bottom surface of the cone configures a curved surface that is slightly convex (semispherical).

The size of the attachment portion body 41 is not particularly limited as long as it is a size that allows the attachment portion body 41 to be attached to the tympanic cavity 81. Considering that the shape of the tympanic cavity 81 varies between individuals, the attachment portion body 41 just needs to be capable of being smoothly attached to most people and remaining attached without being detached by accident after attached. In addition, instead of the ear attachment portion 40 (the attachment portion body 41 or the extending portion 42) having one size, ear attachment portions 40 having multiple sizes may be prepared such that they can be replaced.

A diameter R11 of the bottom surface of the cone as the shape of the attachment portion body 41 can be, for example, set to a range of 16 to 27 mm. In addition, a height H11 of the cone can be set to 8 to 20 mm.

The extending portion 42 has a cylindrical shape and extends from the side surface of the cone as the shape of the attachment portion body 41. The extending portion 42 can extend from a position that is distant from a midpoint of the cone in the height direction toward the apex. One end portion 62 of the connection portion 60 is attached to an extending end portion 42a of the extending portion 42.

The size of the extending portion 42 just needs to be a size that allows the extending portion 42 to extend to the outside or near the outside of the ear 80 (external ear) from the attachment portion body 41 fitting to the tympanic cavity 81 through the intertragic notch 84.

A diameter R12 of the cylindrical shape of the extending portion 42 can be set, for example, to 3 to 15 mm. In addition, regarding the length of the extending portion 42, particularly, a length L11 of a portion positioned outside the

attachment portion body 41 when seen in a top view can be set, for example, to 10 to 50 mm.

The material of the attachment portion 50 is not particularly limited, and various plastics can be used. From the viewpoint of processability and cost, an ABS resin, polypropylene, and polyethylene can be preferably used.

Fig. 18 is a perspective view of an ear attachment portion 40A of another form. The ear attachment portion 40A is a modification example of the ear attachment portion 40 shown in Figs. 17A and 17B. In a state of being attached to the concha (tympanic cavity 81), an attachment portion body 41A has through-holes 48 that connect inside and outside of the concha. Due to such a configuration, even in a state where the attachment portion body 41A is attached to the tympanic cavity 81, external sound is not blocked, and the subject of brain wave measurement and the operator of the device or the like can smoothly communicate with each other.

Fig. 19 shows an ear attachment portion 40B of another form. The ear attachment portion 40B is configured such that a helix 82 or an earlobe 85 of the ear 80 (external ear) is sandwiched therebetween. In the example shown in the drawing, the ear attachment portion 40B has a spring-loaded clip mechanism, and includes a front surface portion 40B1, a back surface portion 40B2 facing the front surface portion 40B1, and a spring 40B3 (torsion spring) disposed between 40B1 and 40B2 and applying force to 40B1 and 40B2 in a direction along which the helix 82 is sandwiched therebetween. For example, one end of the connection portion 60 is attached to the front surface portion 40B1. Note that a magnet-type mechanism, a screw-type mechanism, or the like may be used in addition to the clip mechanism.

Fig. 20 shows an ear attachment portion 40C of another form. The ear attachment portion 40C has a shape hooked around the ear 80 (external ear), which is substantially a C-shaped portion herein. In the example shown in the drawing, attached to ear, the ear attachment portion 40C has a curved shape in which one end portion 40C1 is hooked around the upper side of the helix 82 and curved along the auriculotemporal sulcus on the back side (on the head 99 side), and the other end portion 40C2 reaches the earlobe 85. For example, the one end portion 61 of the connection portion 60 connected to a plate-shaped member 52 is attached to a portion that becomes the uppermost side (that is, the band member 11 side) when the ear attachment portion 40C is attached to ear.

<Attachment portion 50>

Figs. 21A and 21B shows the attachment portion 50. Fig. 21A is a side view, and Fig. 20B is a plan view. The attachment portion 50 is attached to both longitudinal ends of the band outer surface 11b of the band member 11. The attachment portion 50 functions as a fixing portion that attaches the conformance assisting portion 30 to the band member 11, and functions as an adjustment portion that adjusts the way the band member 11 is worn on the head 99.

Specifically, the attachment portion 50 has the plate-shaped member 52 in the form of a long belt, and a lock portion 51 that locks the plate-shaped member 52 at a desired position.

A plurality of teeth 52a with a tip portion extending in the short direction is arranged on one main surface of the plate-shaped member 52. One end of the wire-shaped connection portion 60 is attached to one end portion of the plate-shaped member 52.

The lock portion 51 has a path 54 into which the plate-shaped member 52 can be inserted, and is attached to the band outer surface 11b of the band member 11. The path 54 has a claw 55 and a release portion 56.

The claw 55 is disposed such that the claw 55 is not engaged with the teeth 52a when the plate-shaped member 52 is moved in the direction along which the plate-shaped member 52 is inserted into the path 54 (herein, moved to the left), and that the claw 55 is engaged with the teeth 52a when the plate-shaped member 52 is moved in a direction along which the plate-shaped member 52 is drawn from the path 54 (here, moved to the right).

The release portion 56 is interlocked with the claw 55. Operating the release portion 56 makes it possible to release the claw 55 from the teeth 52a such that the plate-shaped member 52 can move to any of the right or left side. That is, with the attachment portion 50, it is possible to adjust the distance to the ear attachment portion 40 attached to the plate-shaped member 52 through the connection portion 60. As a result, the way the band member 11 is worn can be adjusted.

The material of the attachment portion 50 is not particularly limited, and various plastics can be used. From the viewpoints of processability, cost, and the like, 66 nylon can be preferably used.

As the attachment portion 50, for example, it is possible to adopt a hook-and-loop fastener mechanism, a cam buckle-type mechanism, a buckle-type mechanism, a button-type mechanism, and the like. For example, in a case where the hook-and-loop fastener mechanism is used, a portion of a sheet of belt-shaped hook-and-loop fastener is attached to the band member 11 and folded such that surfaces are joined. A portion formed by making the end portion of the connection portion 60 annular is attached to the folded portion. The folding position is adjusted such that the length of the hook-and-loop fastener extending from the band member 11, that is, the distance between the band member 11 to the ear attachment portion 40 is adjusted.

<Connection portion 60>

The connection portion 60 is configured with a solid line member. One end portion thereof is attached to the ear attachment portion 40 (extending portion 42), and the other end portion thereof is attached to the plate-shaped member 52 of the attachment portion 50.
The material of the connection portion 60 is not particularly limited, and various plastics such as polyvinyl chloride and polyethylene can be used. The connection portion 60 may be configured with a nonstretchable resinous member or may be configured with a stretchable elastic member (rubber-like member). In a case where an elastic member is used as the connection portion 60, due to appropriate tension caused by the elastic force and the length adjusting function of the attachment portion 50, it is possible to create an excellent brain wave measurement environment.

<Method of putting on and adjusting conformance assisting portion 30>

The method of putting on the conformance assisting portion 30 is as follows, for example.
First, for one of the two attachment portions 50, the plate-shaped member 52 is detached from the lock portion 51 or fully stretched, and in this state, the attachment portion body 41 of the ear attachment portion 40 is fitted and fixed to the tympanic cavity 81 (S10).
Subsequently, the connection portion 60, which is a wire, is caused to conform to the back of the ear 80 (S11).
Next, the lock portion 51 of the plate-shaped member 52 is inserted in a state where the connection portion 60 is inhibited from separating from the position to which the connection portion 60 conforms, such that the length of the plate-shaped member 52 extending from the lock portion 51 is adjusted and the plate-shaped member 52 is temporarily fixed (S12).
Furthermore, for the other attachment portion 50, the attachment portion body 41 is attached by the same procedure (S10 to S12), and the length of the plate-shaped member 52 extending from the lock portion 51 is adjusted and the plate-shaped member 52 is temporarily fixed (S13).
After the temporary fixing of both the attachment portions 50 ends, the length of the plate-shaped member 52 extending from the lock portion 51 is finely adjusted, such that the tightness of the band member 11 by the conformance assisting portion 30 is adjusted (S14).
After the adjustment (S14), the brain wave measurement is started.

<Effect of Embodiment>

The aforementioned features and effects of the embodiments are summarized as follows.

(1) The brain wave measuring device 10 has the band member 11 that is worn conforming to the shape of the human head 99,

a plurality of electrode portions 13 that is provided on one surface of the band member 11, and
conformance assisting portions 30 that assist the band member 11 in conforming to the shape of the head 99, in which each of the conformance assisting portions 30 has
the ear attachment portion 40 that is worn on the human ear 80 (external ear),
an attachment portion 50 that is attached to the band member 11, and
the connection portion 60 (connection member) that spans between the ear attachment portion 40 and the attachment portion 50.

The brain wave measuring device 10 can be worn regardless of the shape of the human head 99 on which the device is to be worn. Furthermore, having the conformance assisting portion 30, the brain wave measuring device 10 can appropriately assist the band member 11 in conforming to the shape of the head 99. That is, because the brain wave measuring device 10 adopts a fixing method using the external shape of the tympanic cavity 81 or ear 80 located at the longitudinal end of the band member 11 when the band member 11 is worn on the head 99, it is easy to solve the problem in that the band member 11 does not fully conform to the head 99. In addition, it is possible to prevent the electrode portions 13 from floating above the head 99 due to the reaction force caused by the hair. Moreover, it is possible to prevent the band member 11 from being detached from the head 99 due to everyday movements.
(2) The conformance assisting portion 30 has an adjustment portion that adjusts the way the band member 11 conforms to the head 99.
(3) The adjustment portion has a mechanism that adjusts the distance between the ear attachment portion 40 and the attachment portion 50.
For example, because the attachment portion 50 (the plate-shaped member 52 and the lock portion 51) functions

as the adjustment portion, the brain wave measuring device appropriately conforms to the head, that is, a brain wave measuring environment can be created, regardless of the shape and size of the human head 99 on which the device is worn.

(4) The adjustment portion includes the plate-shaped member 52 and the lock portion 51 that locks the plate-shaped member 52 at a predetermined position.

By adopting a configuration in which the plate-shaped member 52 is inserted into the path 54 of the lock portion 51 and the claw 55 locks the teeth 52a of the plate-shaped member 52, it is possible to simultaneously achieve fine adjustment and appropriate fixing.

(5) The connection portion 60 has an elastic member.

By adopting an elastic member such as rubber wire as the connection portion 60 that connects the attachment portion 50 and the ear attachment portion 40, it is possible to adjust the length and adjust the way the band member 11 (protrusion portions 12) comes into contact with the head 99 in a well-balanced manner.

(6) The ear attachment portion 40 is attached by fitting in the concha (that is, the tympanic cavity 81).

(7) In a state of being attached to the concha (tympanic cavity 81), the ear attachment portion 40 has through-holes 48 that connect inside and outside of the concha.

Even in a case where the ear attachment portion 40 is fitted into the tympanic cavity 81, external sound is not blocked, which enables the subject and the operator to remain in communication with each other during brain wave measurement.

(8) The ear attachment portion 40 is attached by sandwiching the external ear portion.

(9) The ear attachment portion 40 is attached by being hooked around the external ear portion.

(10) The band member 11 is configured with a rubber-like elastic material.

Being a rubber-like elastic material, the band member 11 conforms to the shape of the human head 99. As a result, the brain wave measuring device 10 can be worn regardless of the shape of the human head 99.

(11) The brain wave measuring device 10 has a plurality of elastic protrusion portions 12 integrated with the band member 11, and

the electrode portions 13 are provided on the protrusion portions 12.

By arranging the plurality of the protrusion portions 12 in the entirety of the band member 11, it is possible to disperse pressure when the device is worn on the head. Furthermore, being worn on the head, the brain wave measuring device 10 has flexibility conforming to the shape of the head 99, which prevents pressure from being concentrated on a specific protrusion portion 12 (electrode portion 13) and makes it possible to cause a person to feel discomfort. In other words, it is possible to avoid a phenomenon where discomfort is caused and influences the measurement results. In addition, because the band member 11 is an elastic material having flexibility, the electrode portions 13 of the protrusion portions 12 come into contact with the head 99 in an appropriate direction under appropriate pressure. In this respect, it is also possible to stably detect brain waves.

(12) The electrode portions 13 have conductive members provided on at least the tip portion of the protrusion portions 12.

(13) In the brain wave measuring method, the brain wave measuring device 10 described above is worn on the head 99 of a subject to measure brain waves.

This application claims priority on the basis of Japanese Patent Application No. 2021-094959 filed on June 7, 2021, the entire disclosure of which is incorporated into the present specification.

REFERENCE SIGNS LIST

1 brain wave detection system
10 brain wave detection electrode
11, 11A, 11B, 11C, 11D, 11E, 11F, 11G, 11H, 11I band member
11a band inner surface
11b band outer surface
12 protrusion portion
13 electrode portion
14 signal line
20 brain wave display device
30 conformance assisting portion
40, 40A, 40B, 40C ear attachment portion

41 attachment portion body
42 extending portion
50 attachment portion
51 lock portion
52 plate-shaped member
52a tooth
54 path
55 claw
60 connection portion

## Claims

**1.** A brain wave measuring device comprising:

a band member that is worn on a human head by conforming to a shape of the human head;
a plurality of electrode portions that is provided on one surface of the band member; and
conformance assisting portions that assist the band member in conforming to the shape of the head,
wherein each of the conformance assisting portions has an ear attachment portion that is attached to a human ear, an attachment portion that is attached to the band member, and a connection member that spans between the ear attachment portion and the attachment portion.

**2.** The brain wave measuring device according to claim 1,
wherein each of the conformance assisting portions has an adjustment portion that adjusts the way the band member conforms to the shape of the head.

**3.** The brain wave measuring device according to claim 2,
wherein the adjustment portion has a mechanism that adjusts a distance between the ear attachment portion and the attachment portion.

**4.** The brain wave measuring device according to claim 3,
wherein the adjustment portion has a plate-shaped member and a lock portion that locks the plate-shaped member at a predetermined position.

**5.** The brain wave measuring device according to any one of claims 1 to 4,
wherein the connection member has an elastic member.

**6.** The brain wave measuring device according to any one of claims 1 to 4,
wherein the ear attachment portion is attached to concha by being fitted to the concha.

**7.** The brain wave measuring device according to claim 6,
wherein in a state of being attached to the concha, the ear attachment portion has through-holes that connect inside and outside of the concha.

**8.** The brain wave measuring device according to any one of claims 1 to 4,
wherein the ear attachment portion is attached by sandwiching an external ear portion.

**9.** The brain wave measuring device according to any one of claims 1 to 4,
wherein the ear attachment portion is attached by being hooked around an external ear portion.

**10.** The brain wave measuring device according to any one of claims 1 to 4,
wherein the band member is configured with a rubber-like elastic material.

**11.** The brain wave measuring device according to any one of claims 1 to 4, further comprising:

a plurality of elastic protrusion portions integrated with the band member,
wherein the electrode portions are provided on the protrusion portions.

**12.** The brain wave measuring device according to claim 11,
wherein each of the electrode portions has a conductive member provided on at least a tip portion of each of the protrusion portions.

**13.** A brain wave measuring method for measuring brain waves by putting the brain wave measuring device according to any one of claims 1 to 4 on a subject's head.

FIG. 1

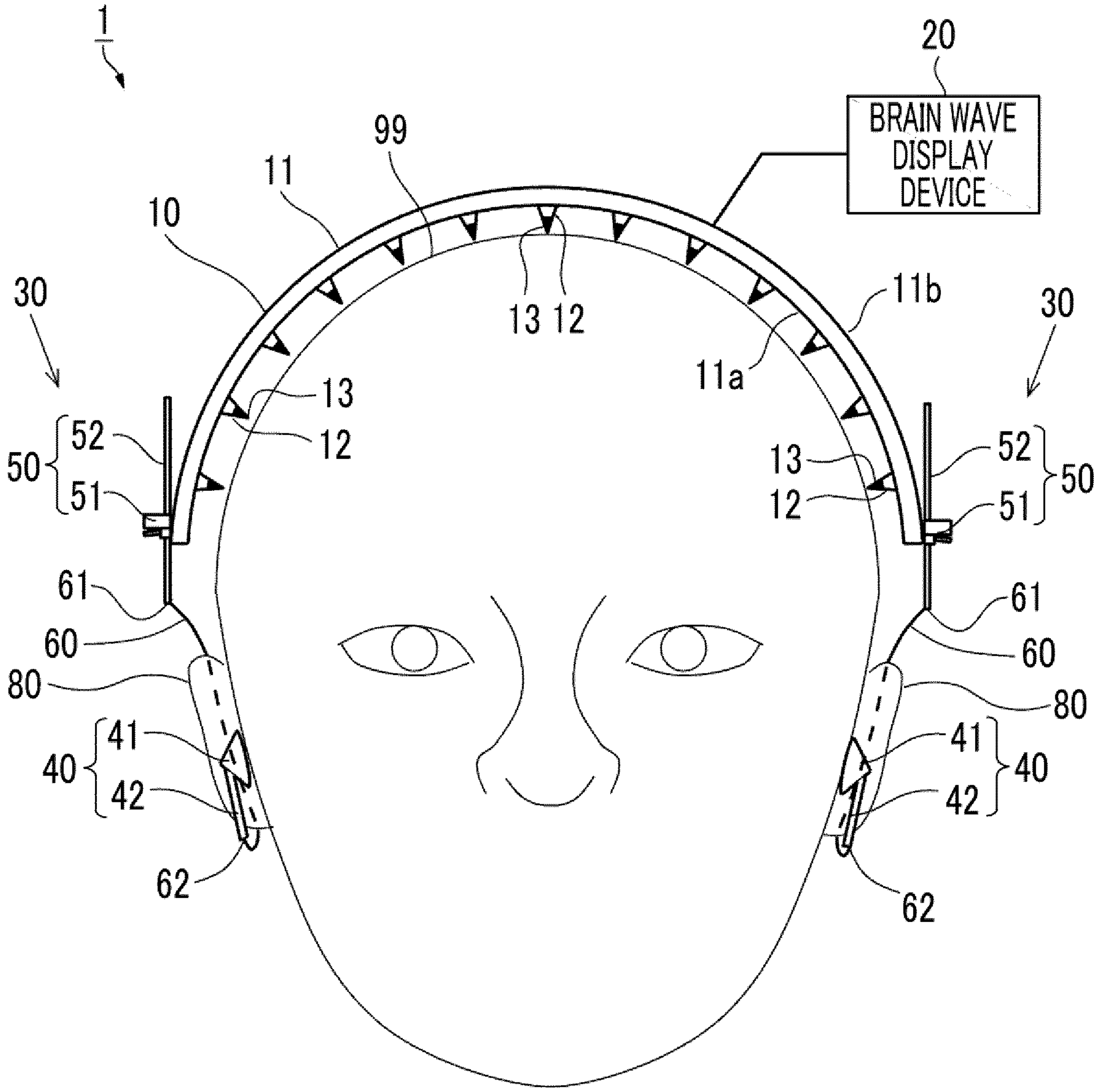
1
20
BRAIN WAVE
DISPLAY
DEVICE
99
11
10
30
13 12
11b
30
11a
13
12
52
50
51
13
12
52
50
51
61
60
80
41
40
42
62
61
60
80
41
40
42
62

FIG. 2

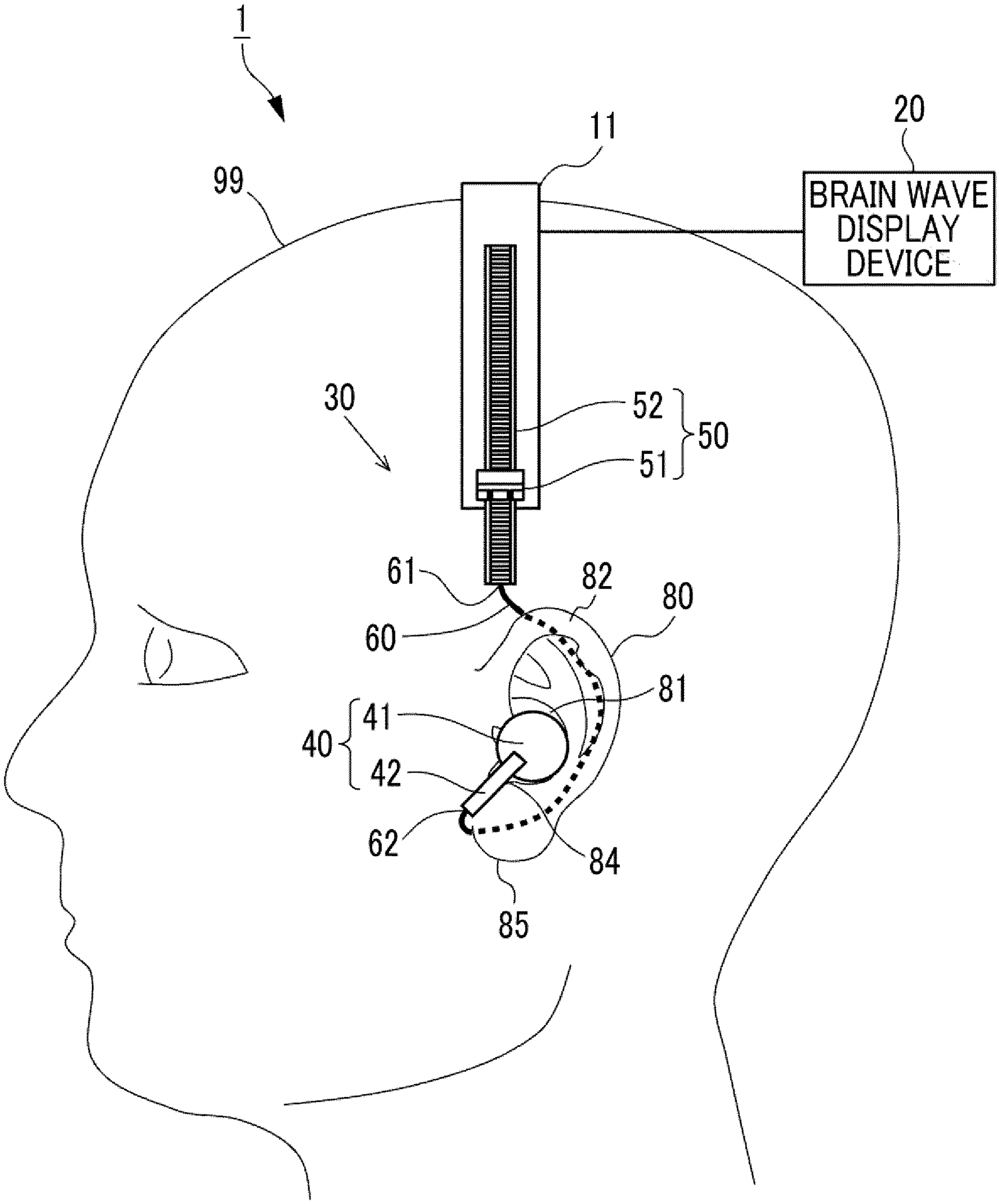
1
11
20
BRAIN WAVE
DISPLAY
DEVICE
99
30
52
51
50
61
82
80
60
81
41
40
42
62
84
85

FIG. 3

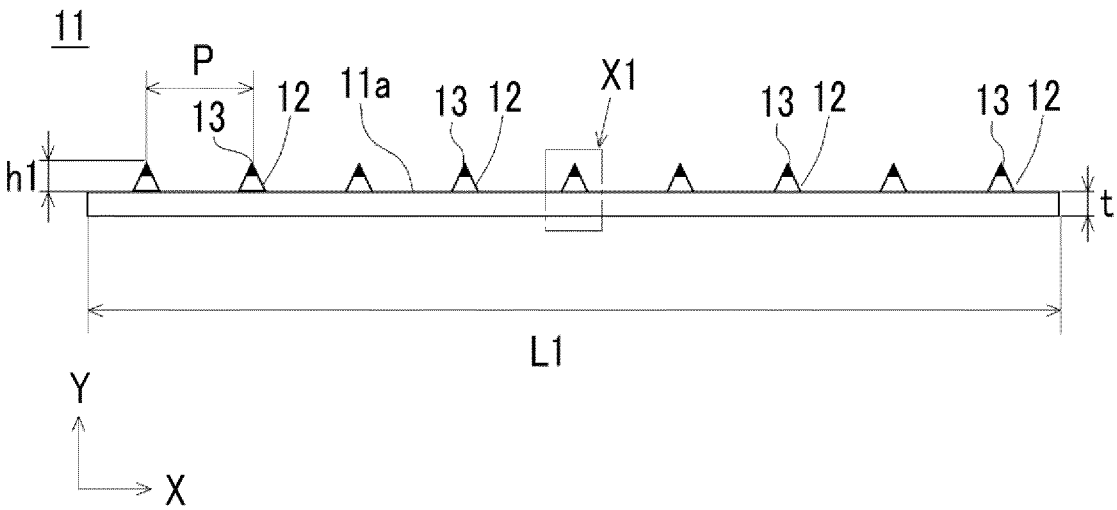
11
P
13
12
11a
13 12
X1
13 12
13 12
h1
t
L1
Y
X

FIG. 4

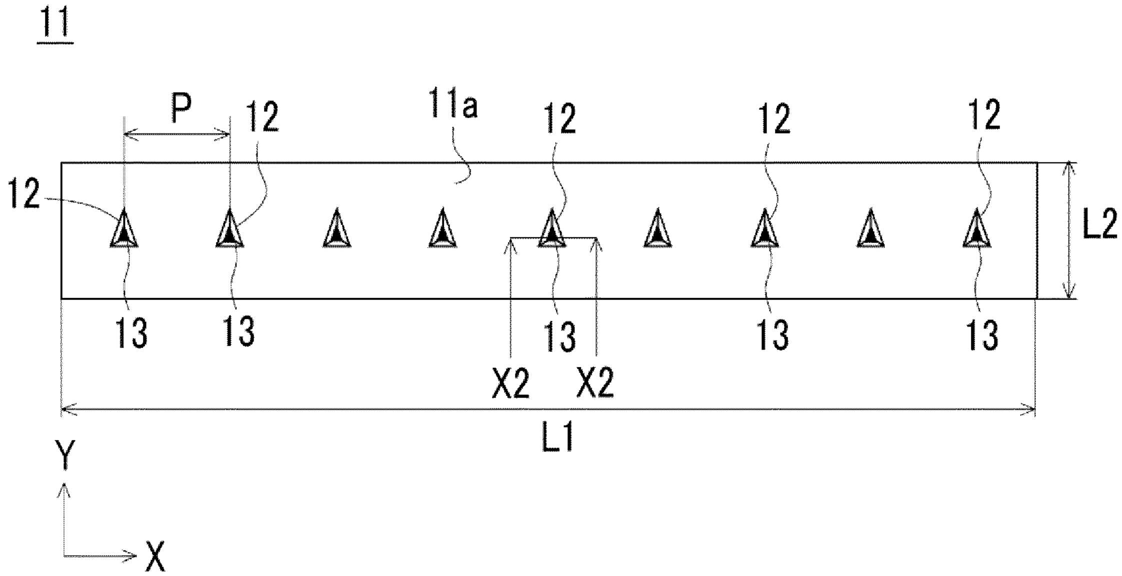
11
P
12
11a
12
12
12
12
L2
13
13
13
13
13
X2
X2
L1
Y
X

FIG. 5

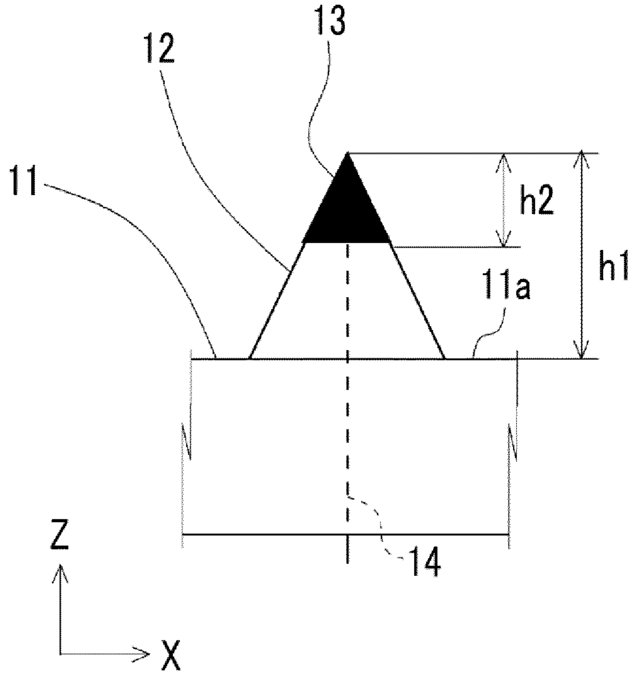


FIG. 6

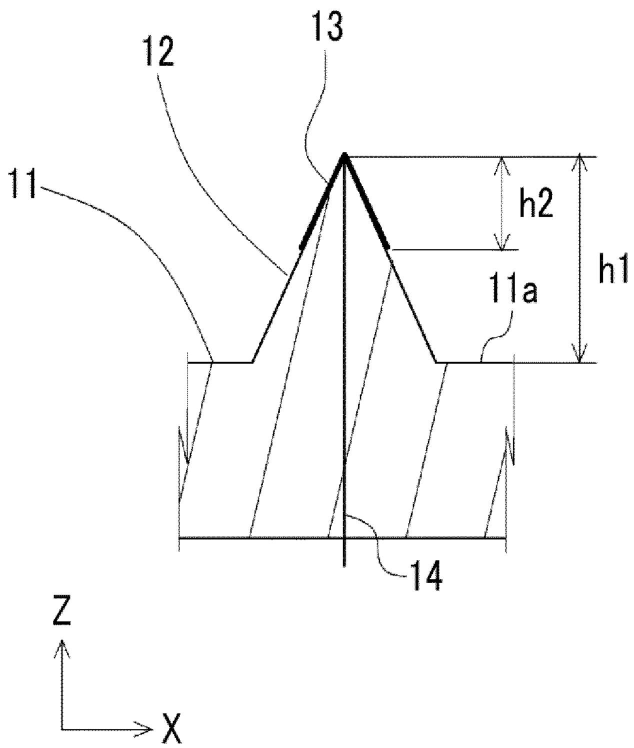

FIG. 7

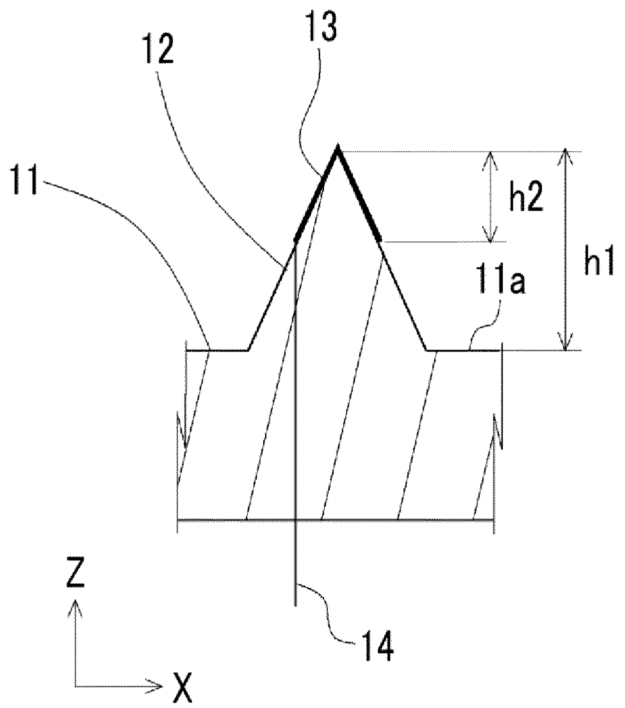


FIG. 8

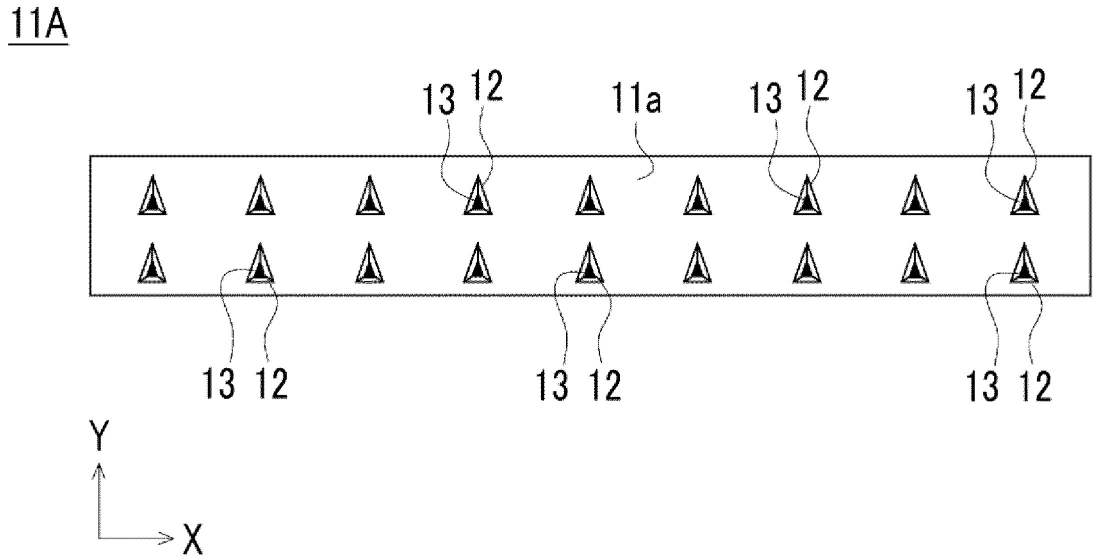

FIG. 9

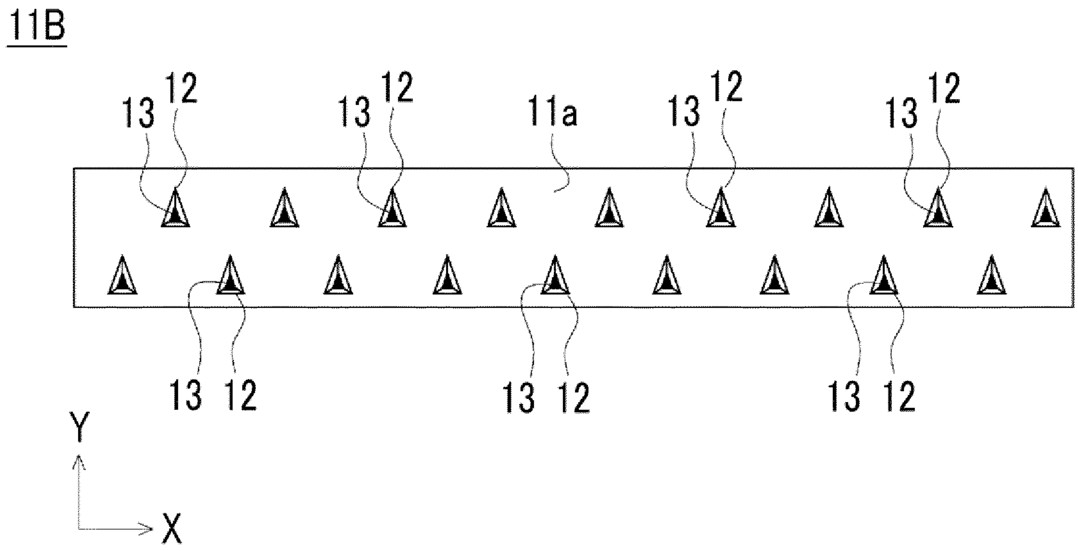


FIG. 10

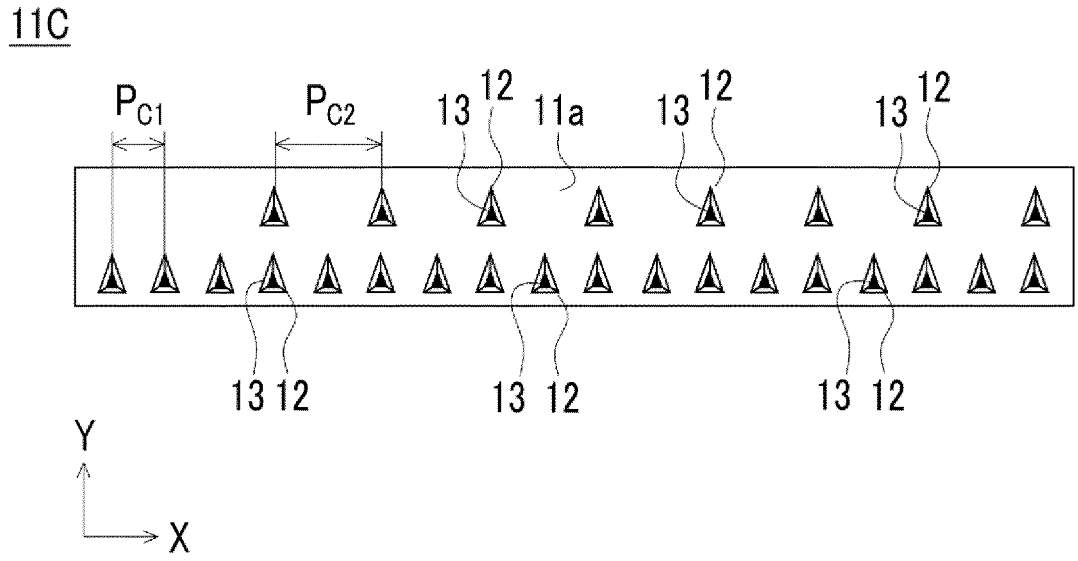

FIG. 11

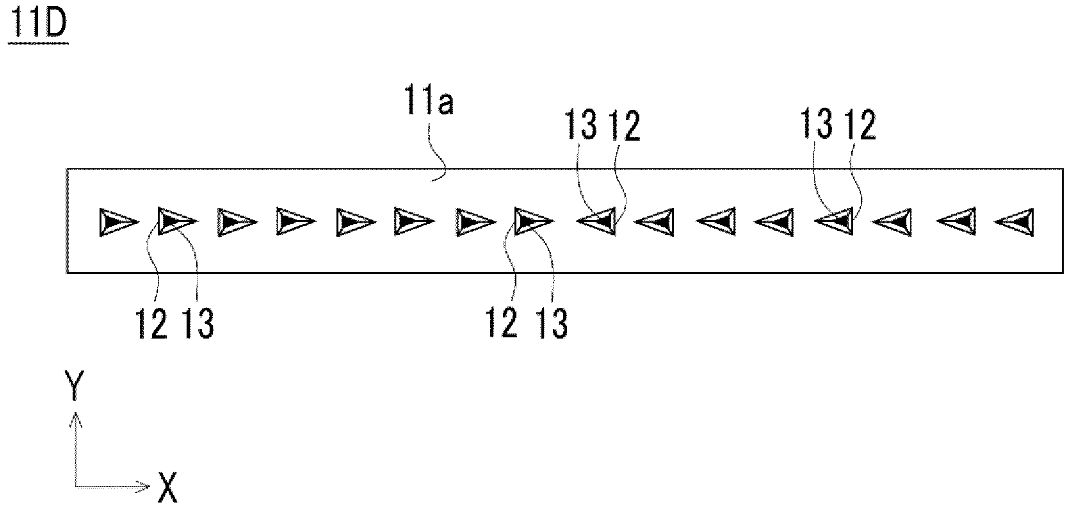


FIG. 12

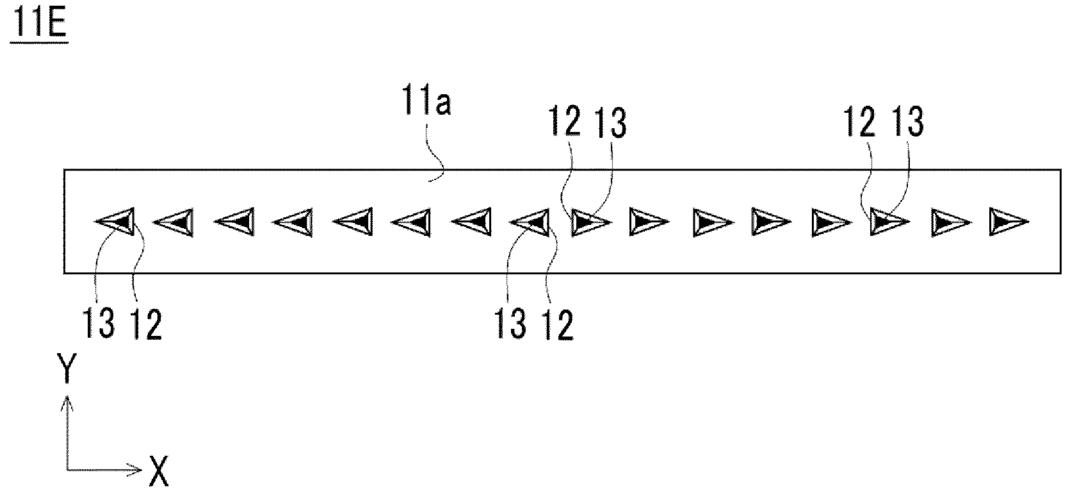

FIG. 13

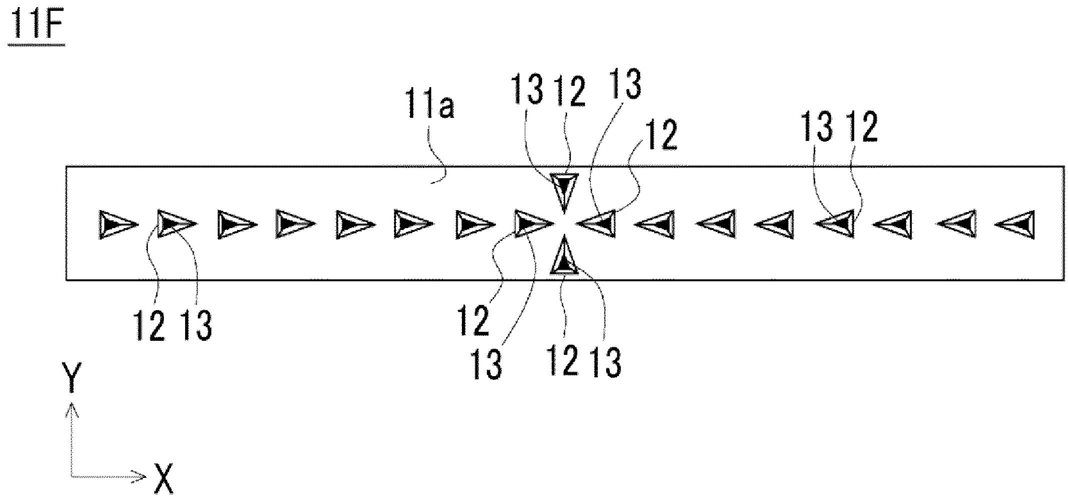
11F
11a
13 12
13
12
13 12
12 13
12
13
12 13
Y
X

FIG. 14

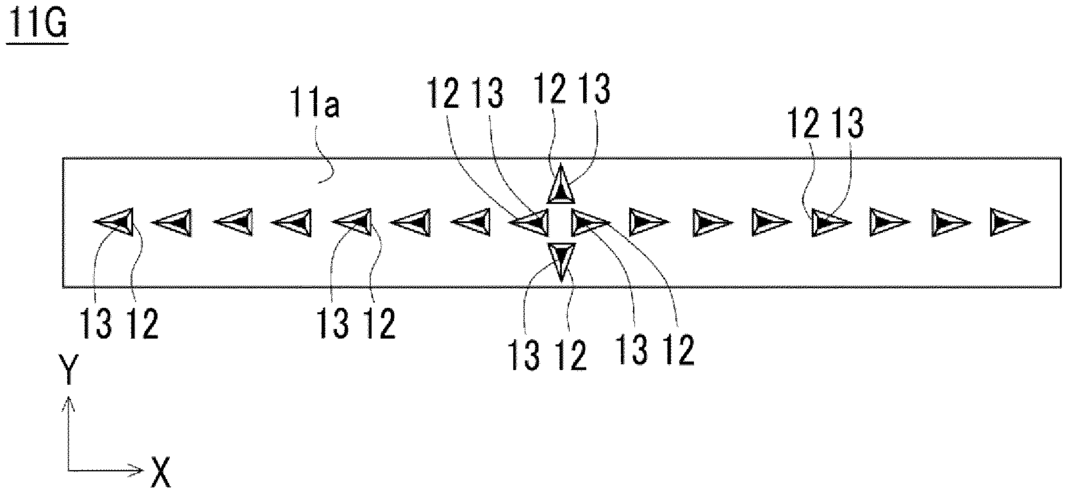
11G
11a
12 13
12 13
12 13
13 12
13 12
13 12
13 12
Y
X

FIG. 15

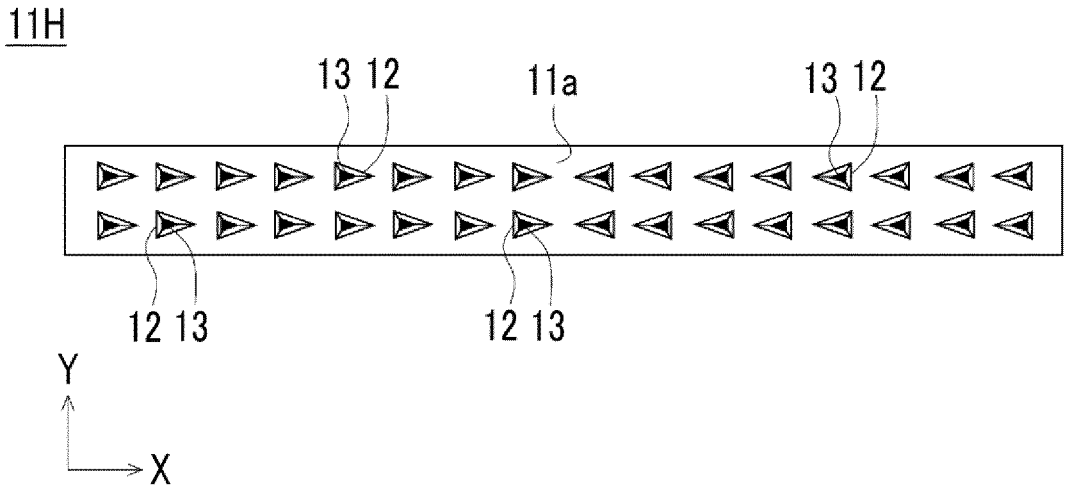


FIG. 16

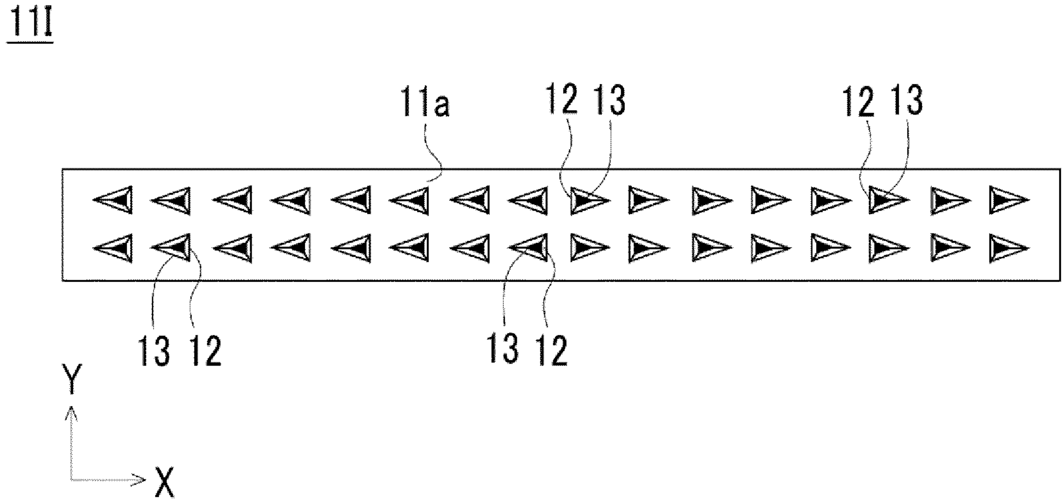

FIG. 17A

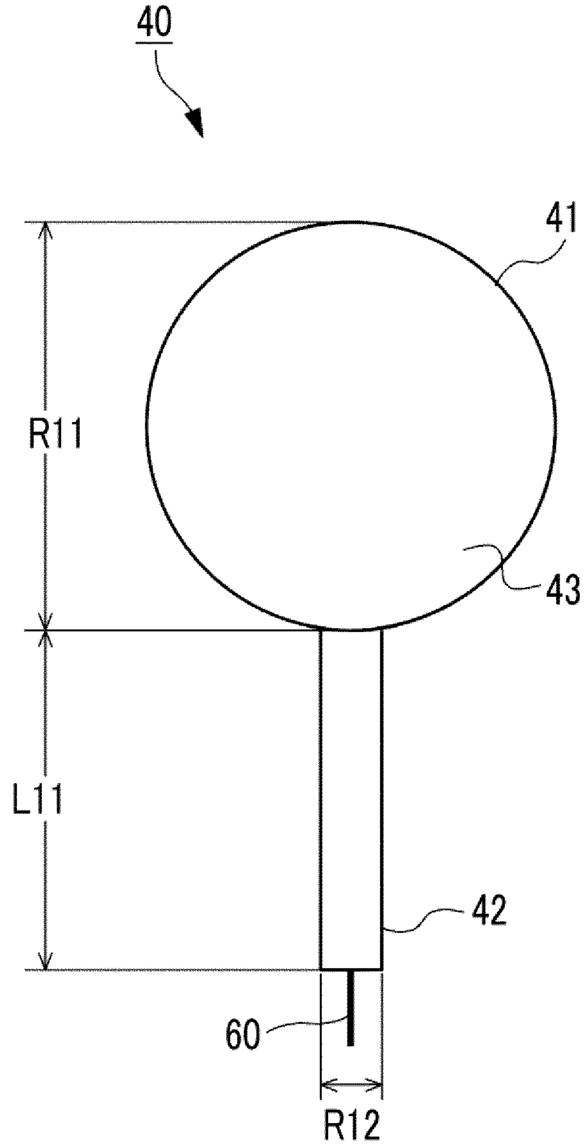


FIG. 17B

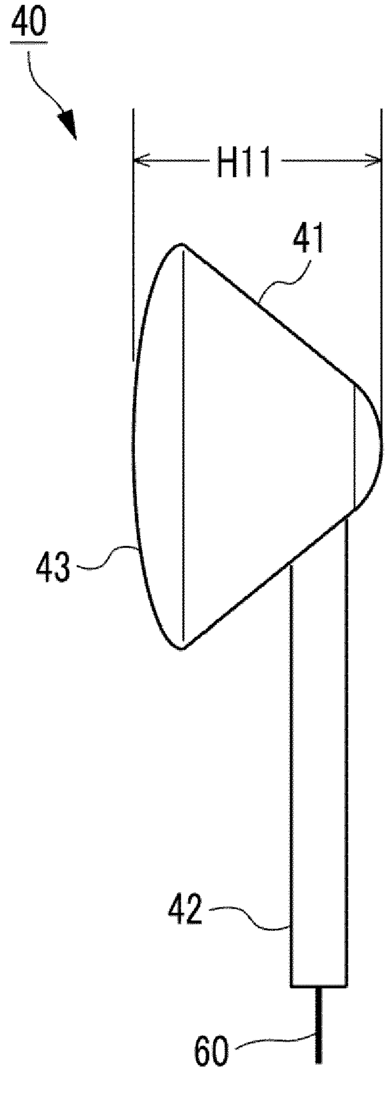

FIG. 18

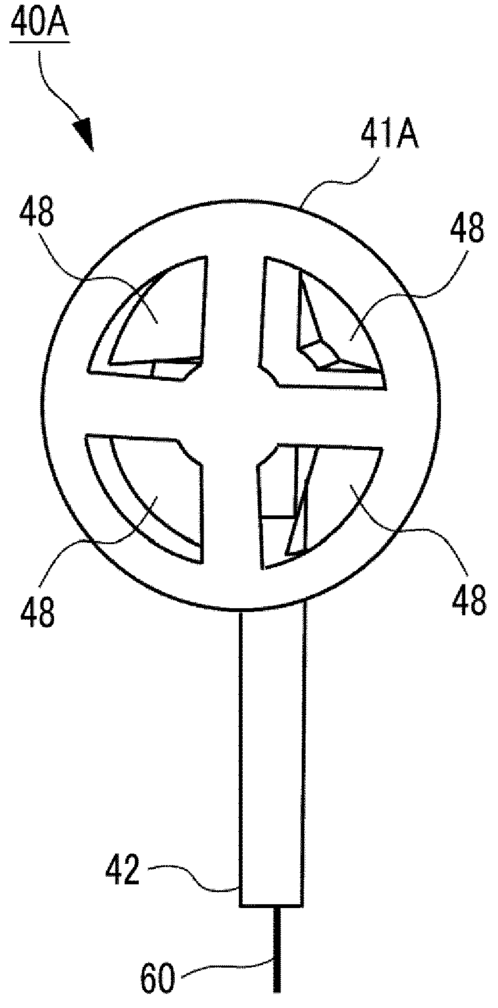

FIG. 19

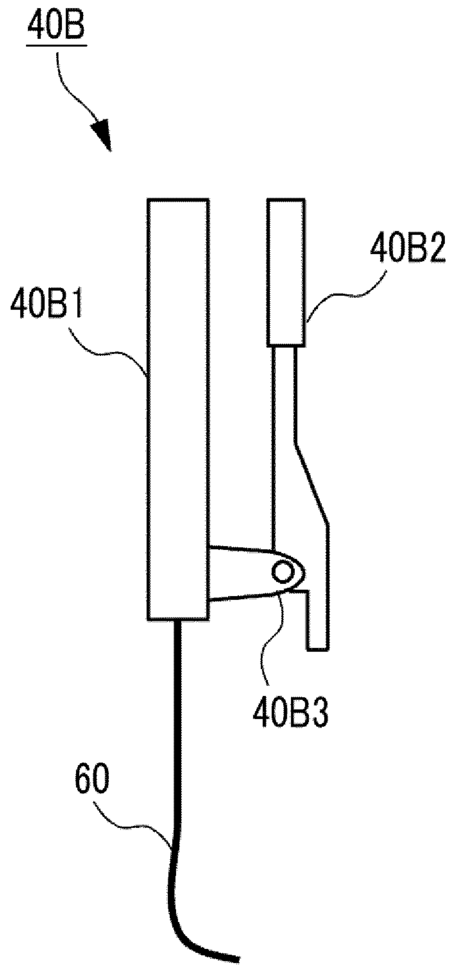

FIG. 20

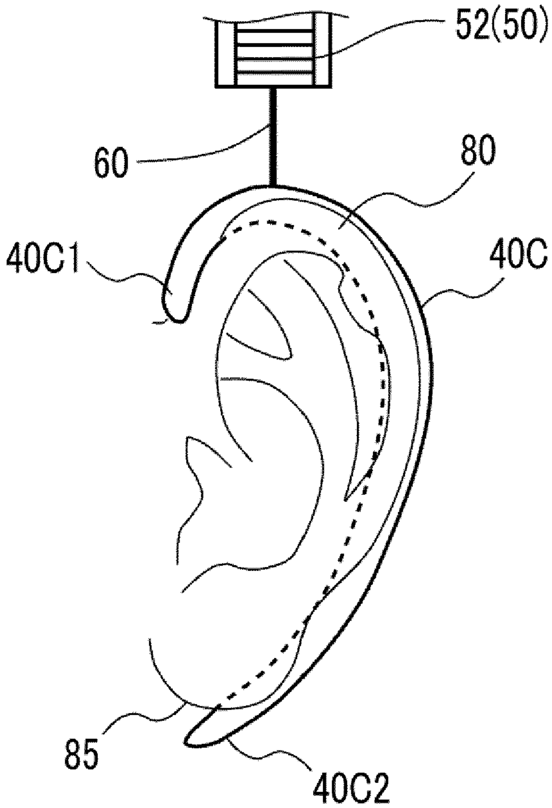

FIG. 21A

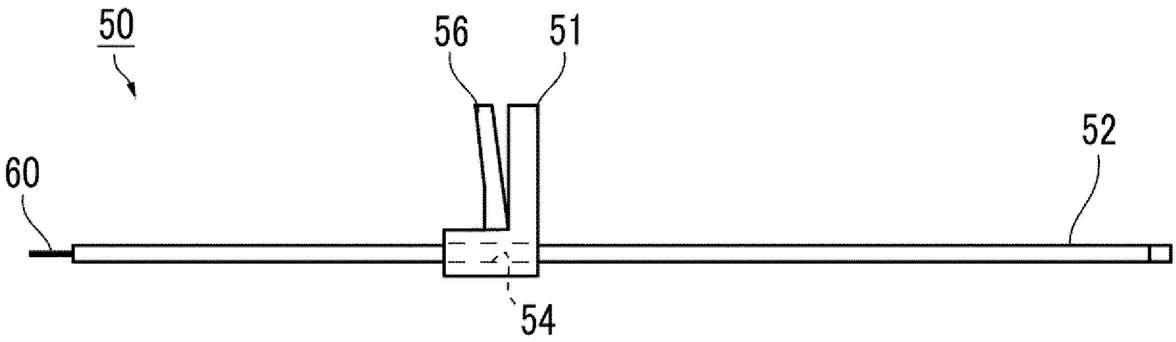


FIG. 21B

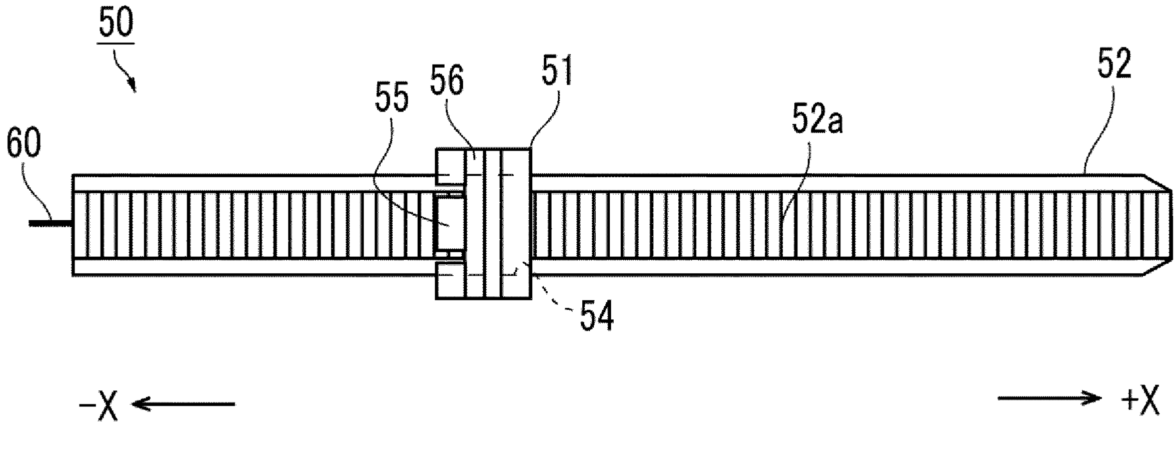

## INTERNATIONAL SEARCH REPORT

International application No. **PCT/JP2022/020716**

**A. CLASSIFICATION OF SUBJECT MATTER**

***A61B 5/256***(2021.01)i
FI: A61B5/256 110

According to International Patent Classification (IPC) or to both national classification and IPC

**B. FIELDS SEARCHED**

Minimum documentation searched (classification system followed by classification symbols)

A61B5/256

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | US 2016/0367189 A1 (INTERAXON INC.) 22 December 2016 (2016-12-22) paragraphs [0032]-[0123] | 1-13 |
| A | US 2019/0223747 A1 (CHOU, Changan) 25 July 2019 (2019-07-25) entire text, all drawings | 1-13 |
| A | CN 205697764 U (CHOU, Changan) 23 November 2016 (2016-11-23) entire text, all drawings | 1-13 |

☐ Further documents are listed in the continuation of Box C.

☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **20 July 2022** | **02 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

**INTERNATIONAL SEARCH REPORT**
**Information on patent family members**

International application No.
**PCT/JP2022/020716**

| Patent document cited in search report | Publication date (day/month/year) | Patent family member(s) | Publication date (day/month/year) |
|---|---|---|---|
| US 2016/0367189 A1 | 22 December 2016 | WO 2015/100499 A1<br>paragraphs [0032]-[0122] | |
| US 2019/0223747 A1 | 25 July 2019 | WO 2017/125082 A1<br>entire text, all drawings<br>CN 205899176 U | |
| CN 205697764 U | 23 November 2016 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018175287 A
- JP 2018094434 A
- JP 2021094959 A